# EUROPEAN PATENT APPLICATION

(11) **EP 4 585 612 A1**
(43) Date of publication of application: **16.07.2025**
(21) Application number: 24382029.7
(22) Date of filing: 12.01.2024
(51) Int. Cl.: C07K 14/725, A61K 39/00, A61K 51/10, C07K 16/28, C12N 15/62

(54) **FUSION PROTEINS CONTAINING CITOMEGALOVIRUS US6 PROTEIN**

(71) Applicant: Fundació de Recerca Clínic Barcelona-Institut d'Investigacions Biomèdiques August Pi I Sunyer, 08036 Barcelona (ES); Hospital Clínic de Barcelona, 08036 Barcelona (ES)
(72) Inventor: VAZQUEZ PORTERO, Mario, 08036 Barcelona (ES); CALDERÓN, Hugo, 08036 Barcelona (ES); JUAN OTERO, Manel, 08036 Barcelona (ES)
(74) Representative: Hoffmann Eitle

(57) **Abstract**

The present invention provides a fusion protein suitable to reduce graft-versus-host and host-versus-graft reactions in allogenic transplants. Said fusion protein comprises an anti-CD3 targeting moiety and a human cytomegalovirus US6 protein.

## Description

### TECHNICAL FIELD

The present invention relates to the field immunological therapy in general; and in particular, provides novel fusion proteins to reduce graft-versus-host and host-versus-graft reactions in allogenic transplants.

### BACKGROUND ART

The human leukocyte antigen (HLA) system is a group of related proteins that are encoded by the major histocompatibility complex (MHC) gene in humans. HLA mismatch leads to the activation of alloreactive T cells, which can cause acute cellular rejection (ACR) after a transplantation. The T-cell receptor (TCR)-CD3 complex mediates recognition of antigenic peptides bound to MHC molecules (pMHC), where the CD3 molecules transduce activation signals to the T cell. Therefore, TCR-HLA interaction is key in the pathogenesis of graft-versus-host disease (GVHD) during the Allogeneic hematopoietic cell transplantation (allo-HCT) and allogeneic Chimeric antigen receptor T (CAR-T) cell therapy.

CAR-T cell therapy leads to extraordinary achievements in antitumor treatments where it leads to remarkable, long-term antineoplastic effects with higher target specificity. Nevertheless, some limitations persist in autologous CAR-T cell therapy, such as high costs, long manufacturing periods, and restricted cell sources. The development of an allogenic universal CAR-T (uCAR-T) cell therapy is an attractive breakthrough point that may overcome most of these drawbacks.

To generate universal CAR-T cells for safe allogenic infusion and therapeutic purpose, it is advisable to effectively block graft-versus-host disease (GVHD) by disrupting the TCR. In addition, it is necessary to suppress HLA expression on the allogeneic T cell to reduce rejection of the recipient immune system.

Human citomegalovirus (HCMV) US glycoproteins are able to hijack the ER-associated degradation (ERAD) machinery to suppress MHC molecules mediated virus antigen presentation and thus escape the immune surveillance system. ER-associated degradation (ERAD) is a protein clearance system. Misfolded, misassembled, or metabolically regulated proteins in the ER are selectively dislocated from the ER into the cytosol via specific membrane penetration machinery and subsequently degraded by the cytosolic ubiquitin proteasome system (UPS).

Both HCMV US2 and US11 are ER resident type I integral membrane glycoproteins, that co-opt this ERAD pathway to promote degradation of the MHC class I heavy chain and hence inhibit MHC class I antigen presentation.

Instead promoting degradation of MHC protein, US3 glycoprotein binds physically to peptide-loaded MHC class I heterodimers which causes the retention of class I complexes in the ER and inhibits the association of invariant chains with class II DR-αβ dimers in the ER, causing the mislocalization of class II complexes and reduced peptide loading

In addition to MHC I molecules, US2 and US3 glycoprotein inhibit class II antigen presentation by destroying or abolishing the functions of class II proteins

HCMV US10 encodes an ER membrane glycoprotein that also interacts with constituents of MHC class I antigen presentation. US10 binds free class I heavy chains and delays their transport from the ER.

Unlike US2, US3, US10, and US11, US6 affects antigen presentation by a different strategy: US6 inhibits the translocation of cytosolic peptides by the TAP complex (TAP1/2). US6 binds to the ER luminal side of TAP1 and causes a conformational change that prevents the binding of ATP. Residues 89-108 in the ER-luminal domain of US6 contribute to binding of US6 to TAP and are sufficient and necessary for this inhibition. Thus, US6 protein can reducing the expression of MHC-I on the surface of the cell.

The present invention proposes a new approach to generate universal T cells using US6 protein.

### BRIEF DESCRIPTION OF DRAWINGS

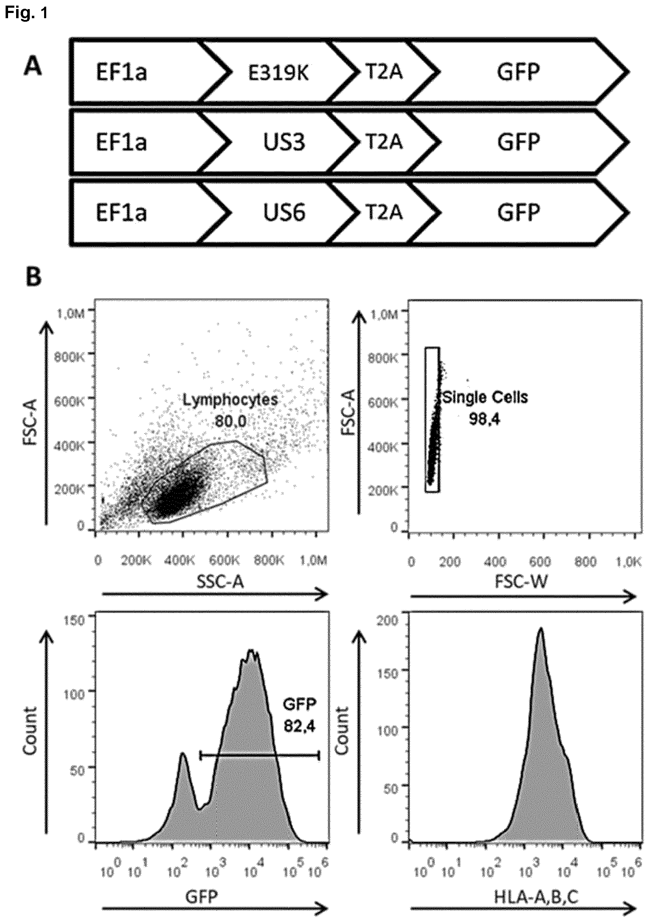

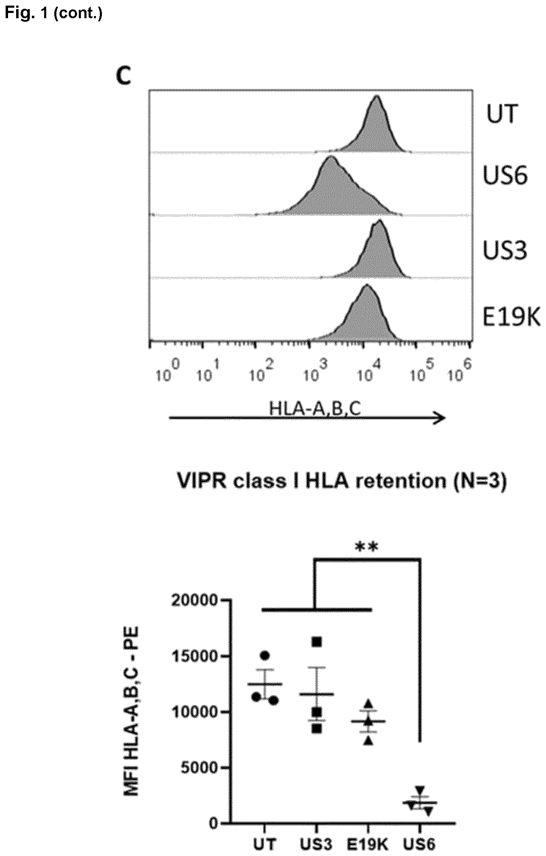

**Fig. 1****:** Evaluation of E19K, US3 and US6 viral proteins to hijack the expression of HLA-I from the surface of human T cells. A) Schematic representation of the DNA constructs cloned into our LV expression vector. B) Gating strategy for the analysis of HLA-I expression in human T cells. C) HLA-I expression on human T cells expressing US3, US6 and E19K proteins from three independent donors.

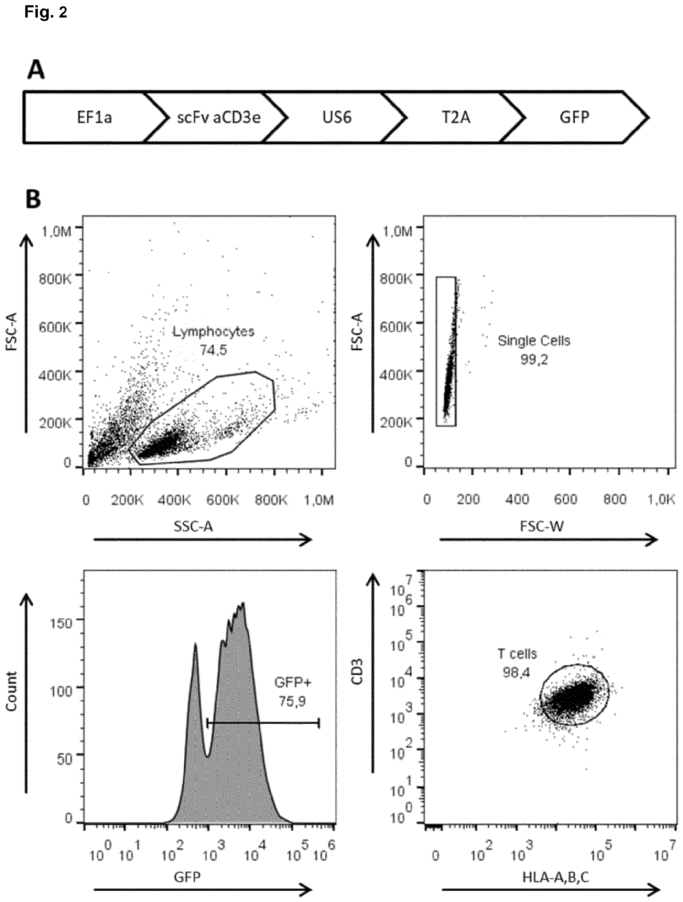

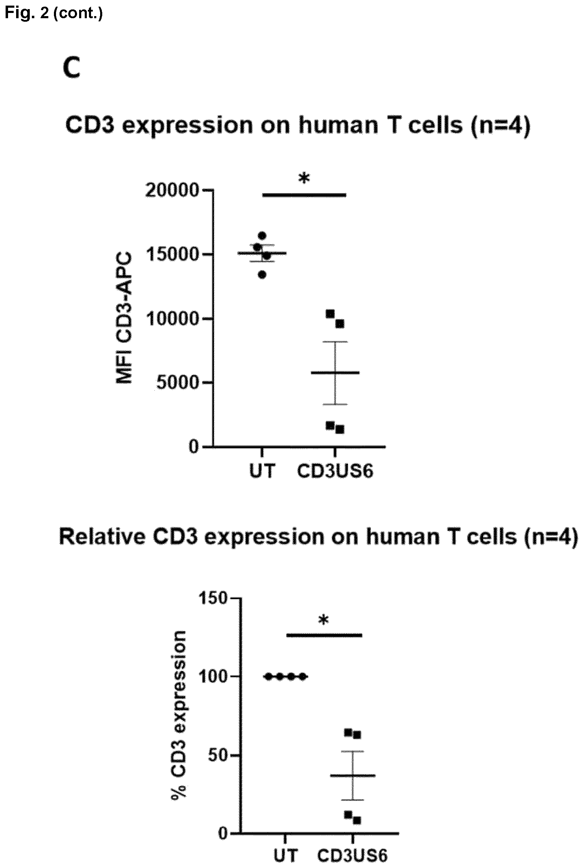

**Fig. 2****:** Evaluation of the CD3 and HLA-I dual retention system in T cells. A) Schematic representation of the transgenic system for the expression of CD3 scFv-US6 chimeric proteins (CD3US6) and the reporter GFP gene. B) Gating strategy for the analysis of CD3 expression in human T cells. C) CD3 expression on human T cells encoding the anti-CD3e-US6 chimeric proteins.

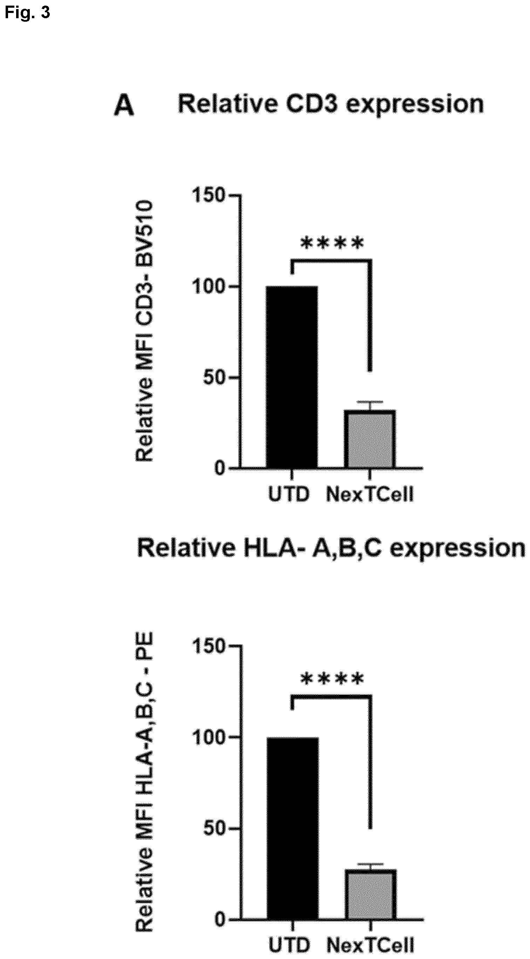

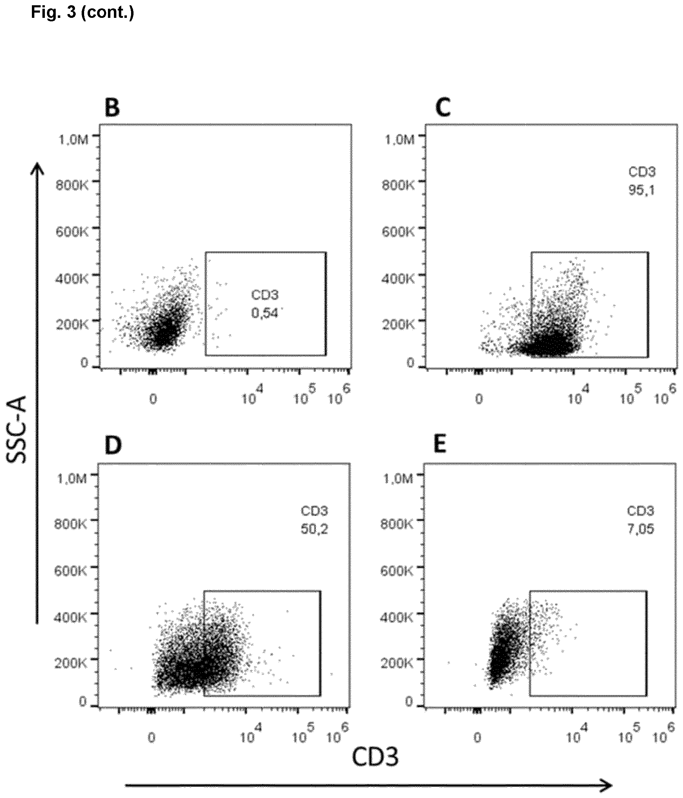

**Fig. 3:** A) CD3 and HLA-A, B, C expression on human T cells encoding the CD3e-US6 chimeric protein. Shows a representation of CD3 expression in a flow cytometry chart of B) Unstained human T cells. C) Untransduced human T cells D) Human T cells expressing anti-CD3e-US6 chimeric protein E) Human T cells expressing anti-CD3e-US6 chimeric protein after magnetic CD3 depletion.

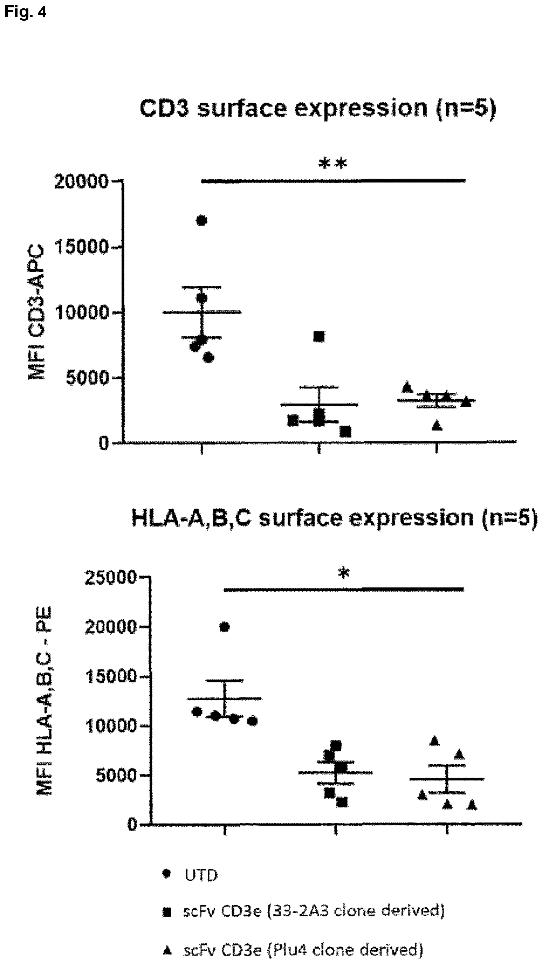

**Fig. 4****:** CD3 and HLA class-I retention with a second anti-CD3e-US6 chimeric protein (also referred herein CD3US6 chimeric protein).

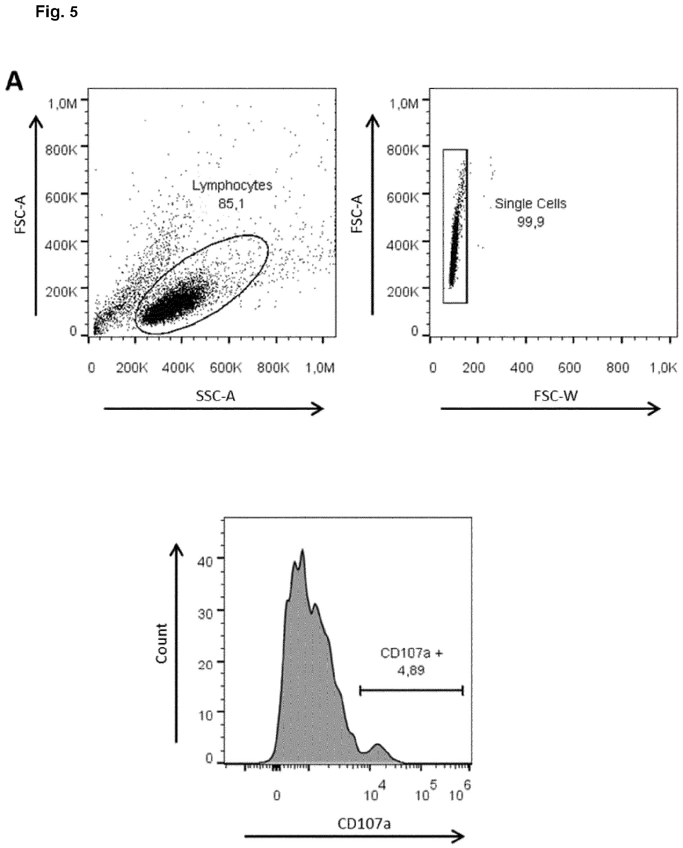

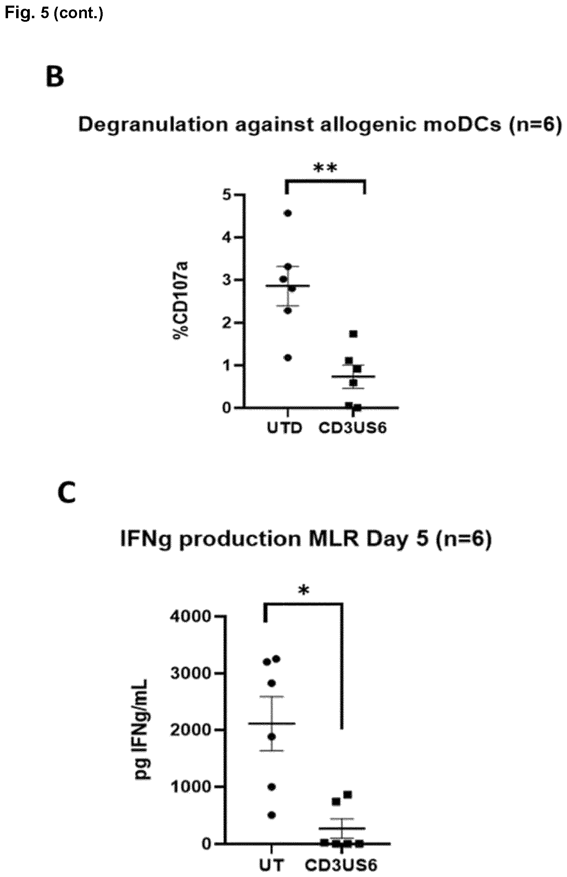

**Fig. 5****:** Allorecognition evaluation of NexTCells on allogenic monocyte derived dendritic cells. A) Representation of gating strategy to evaluate human T cells degranulation against moDCs. B) Graph showing degranulation of UTD and NexTCells against moDCs. C) Graph showing IFNg production of UTD and NexTCells after 5 days of allogenic co-culture.

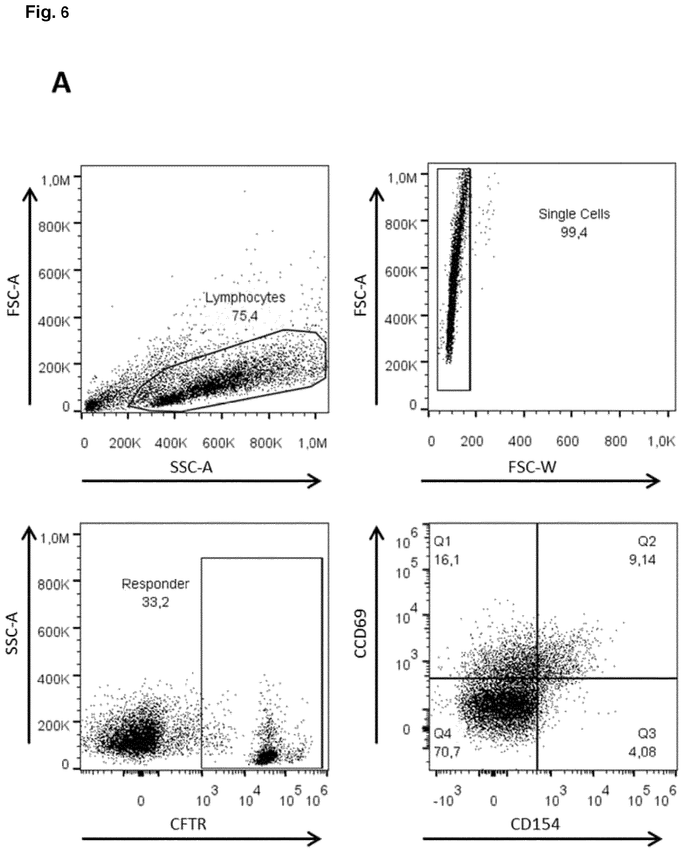

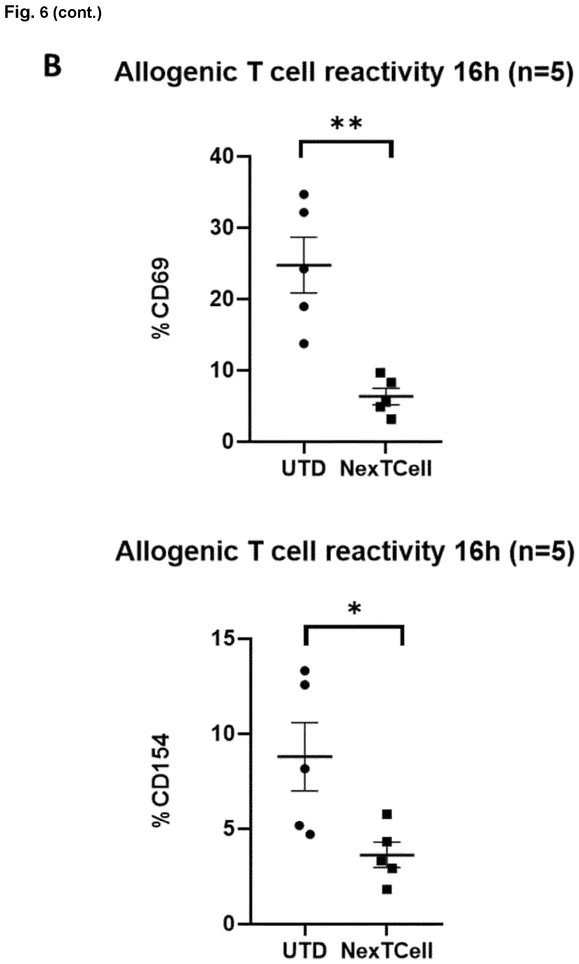

**Fig. 6****:** In vitro rejection evaluation experiment with NexTCells. A) Flow cytometry gating strategy to evaluate human T cells immunogenicity on an allogenic context. B) Graphs showing expression of activation markers on human T cells when co-cultured with allogenic UTD or anti-CD3eUS6 human T cells.

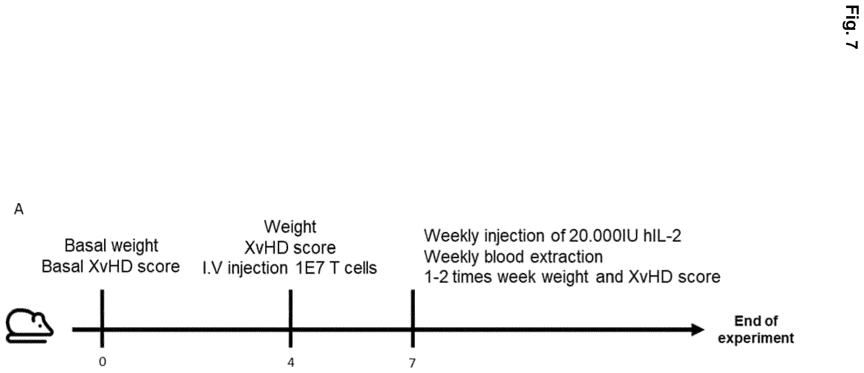

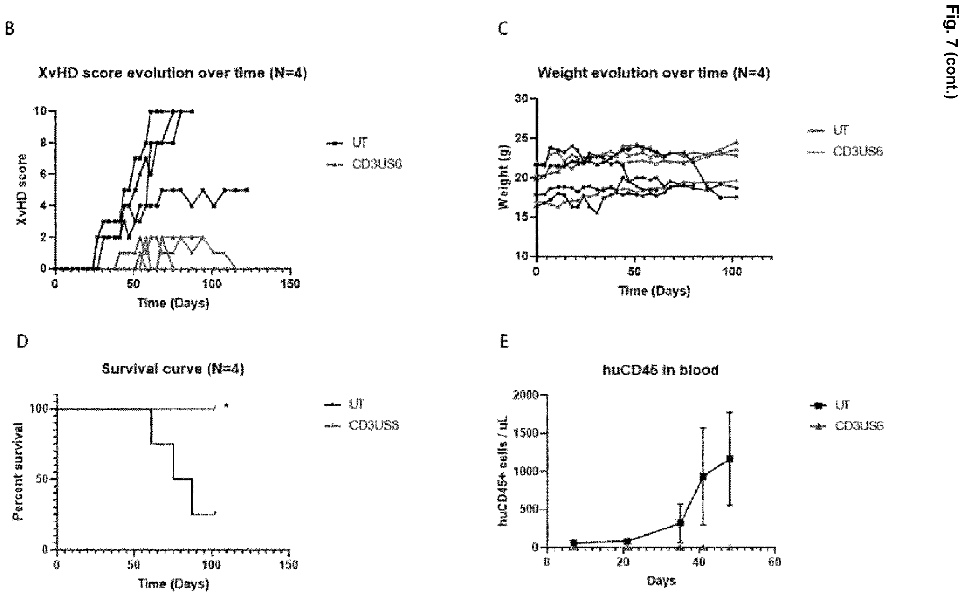

**Fig. 7****:** Evaluation of NexTcells in an in vivo xenograft versus host disease model. A) Timeline of the in vivo experiment. Basal measurements were taken during the first 4 days. After intravenous injection of T cells, mice were monitored at least once a week until the end of the experiment. B) Graph showing individualized xenograft versus host disease scores over time. C) Graph showing individualized weight evolution over time. D) Graphical representation of mice injected with UT or anti-CD3e-US6 T cells survival during the experiment. E) Representation of human T cell proliferation over time in mice blood, quantified as human CD45+ events in flow cytometry.

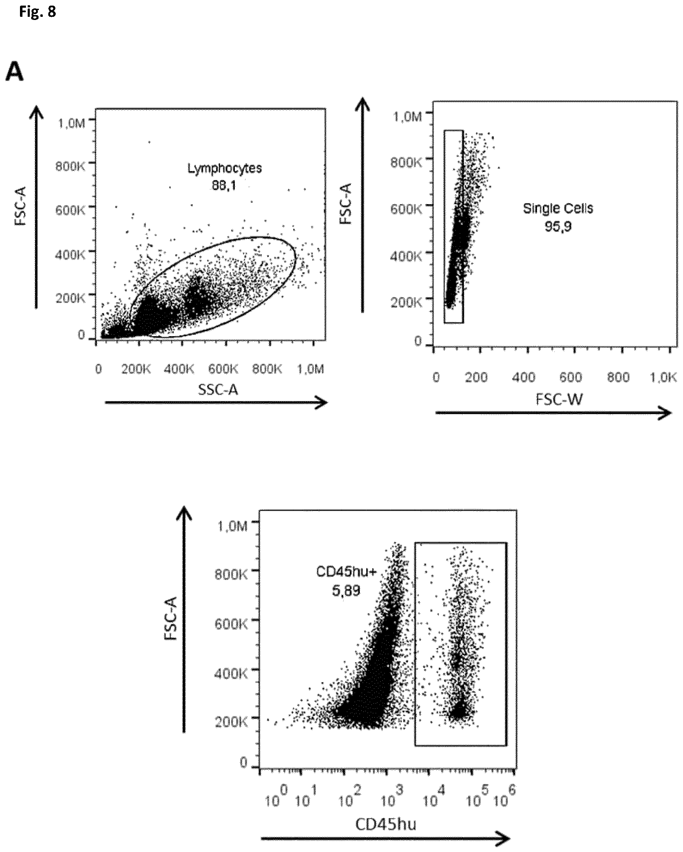

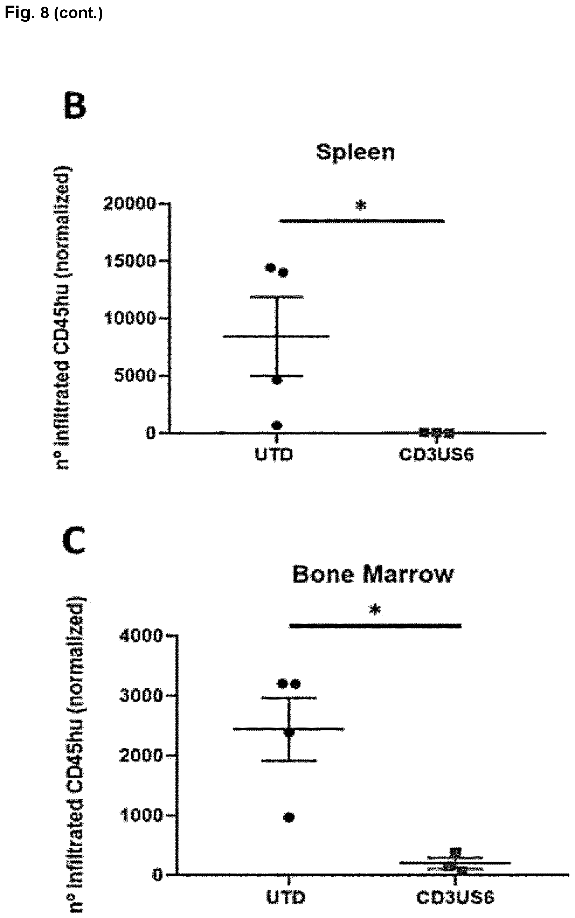

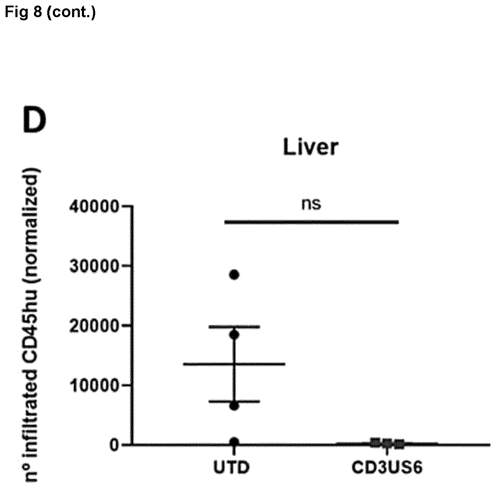

**Fig. 8****:** *In vivo* evaluation of the biodistribution of nexTcell in XxHD model. A) Gating strategy for determining the number of infiltrating human T cells by CD45hu staining in spleen, bone marrow or liver. B), C) and D) Graphs showing number of CD45 human events (normalized) in Spleen, Bone Marrow and Liver, respectively. Organs could not be harvested from one antiCD3e-US6 group mice.

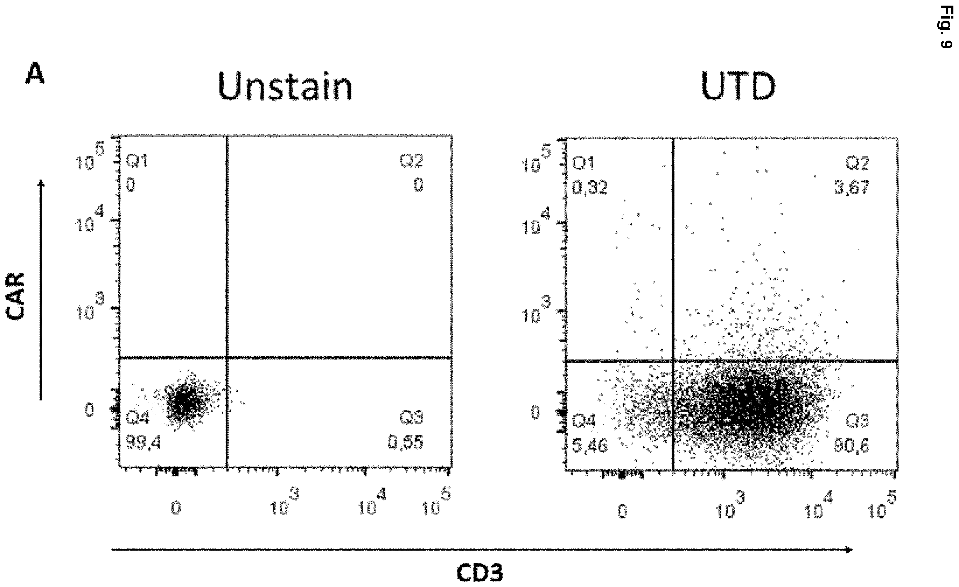

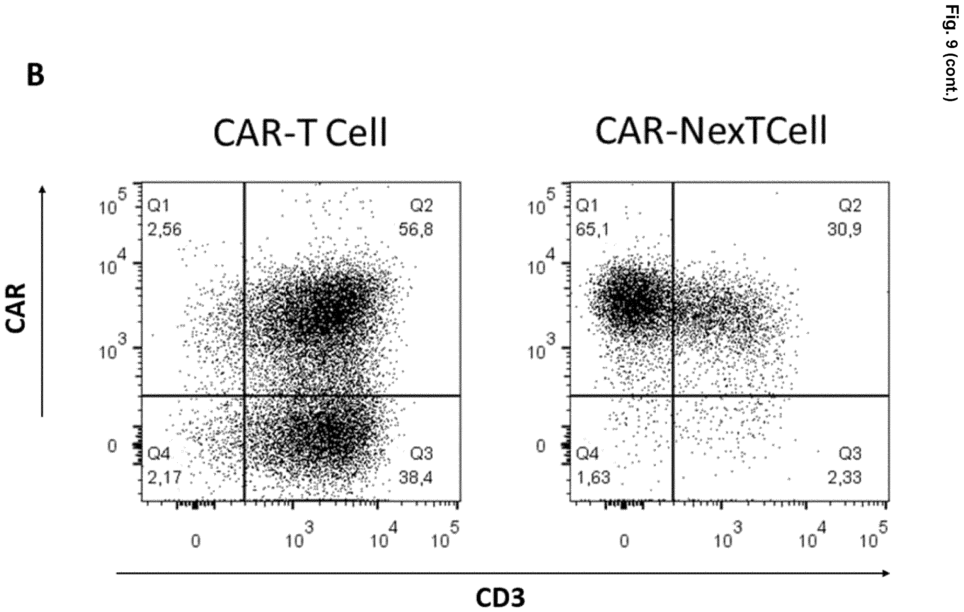

**Fig. 9****:** Figure showing CD3 and CAR expression of human T cells either un-transuced (UTD) (A) or transduced with anti-BCMA CAR transgene (CAR-T cells) or (B) with both anti-BCMA CAR and modified anti-CD3e-US6 transgenes (CAR-NexTCells).

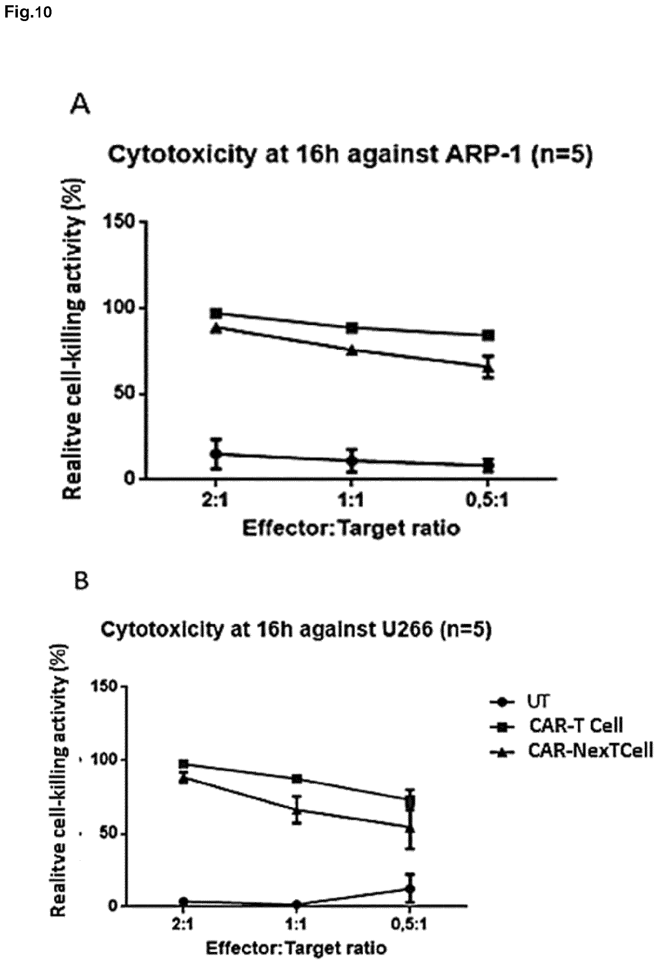

**Fig. 10****:** Comparing CAR-NexTCell to conventional CAR-T cells for their killing activity on myeloma cells expressing BCMA antigen. Graph shows cytotoxicity at 16 hours of untransduced (circles), CAR-T cells (squares) and CAR-NexTCells (triangles) against A) ARP-1 and B) U266 multiple myeloma cell lines.

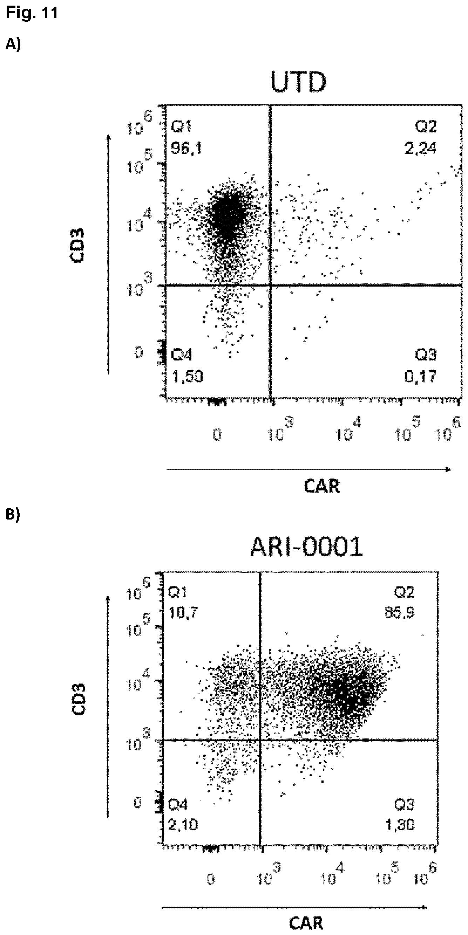

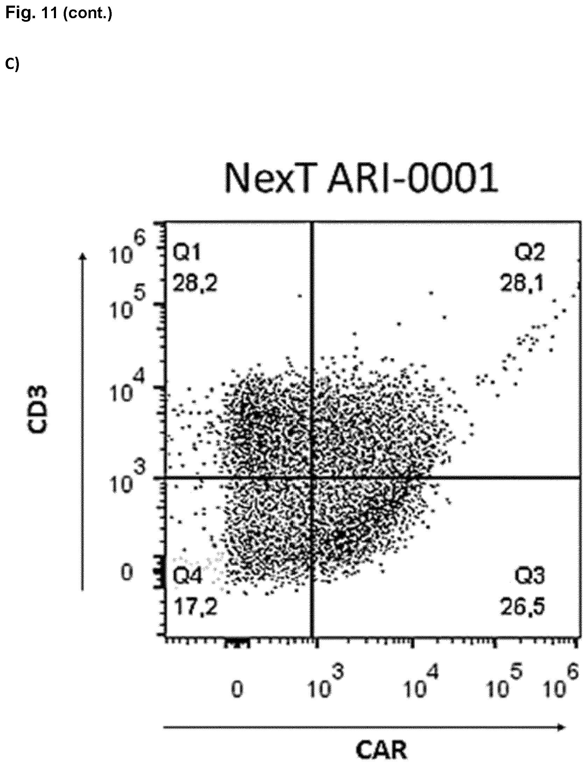

**Fig. 11****:** Assessment on CD3 and CAR expression by flow cytometry. A) UNT: untransduced, B) ARI-0001 CAR and C) combination of ARI-0001 CAR with NexTcell.

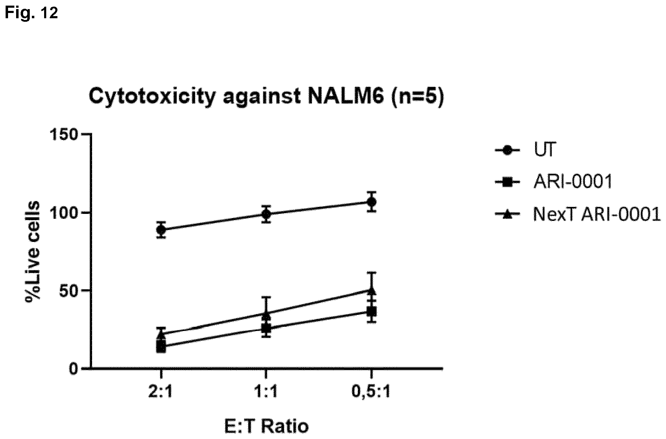

**Fig. 12****:** Cytotoxicity evaluation of ARI-0001 NexTcell against NALM6 (CD19+) cells. Cytotoxic assay comparing ARI-0001 and ARI-0001 NexTcell ARI-0001 cells against NALM6 cell line at different effector to target ratios

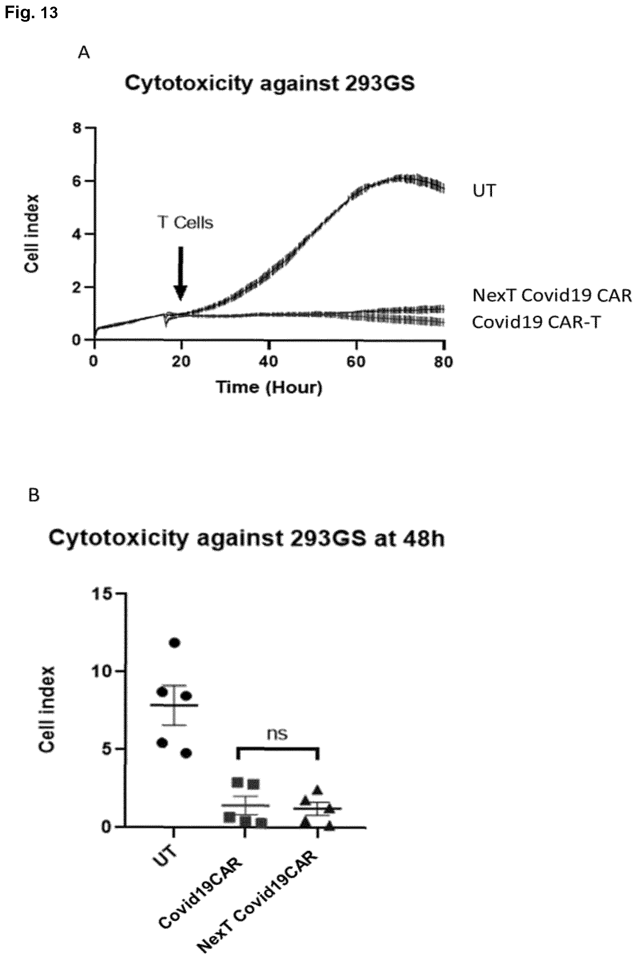

**Fig. 13****:** Cytotoxicity evaluation of Covid19-CAR NexTcell against 293-Spike. A) Live cytotoxicity curves for COVID19-CAR and Covid19-CAR NexTcell against 293-Spike cells assessed by xCELLigence System. B) Cell index comparison of UT, Covid19-CAR and Covid19-CAR NexTcell Covid19-CAR

### GENERAL DEFINITIONS

It must be noted that, as used herein, the singular forms "a", "an", and "the", include plural references unless the context clearly indicates otherwise. Further, unless otherwise indicated, the term "at least" preceding a series of elements is to be understood to refer to every element in the series. Those skilled in the art will recognize or be able to ascertain using no more than routine experimentation, many equivalents to the specific embodiments of the invention described herein. Such equivalents are intended to be encompassed by the present invention.

It is noted that the term "about", as used herein, refers to +/- 30%, preferably +/- 20% or +/- 15%, more preferably +/- 10%, of the indicated referred value.

As used herein, the conjunctive term "and/or" between multiple recited elements is understood as encompassing both individual and combined options. For instance, where two elements are conjoined by "and/or", a first option refers to the applicability of the first element without the second. A second option refers to the applicability of the second element without the first. A third option refers to the applicability of the first and second elements together. Any one of these options is understood to fall within the meaning, and therefore satisfy the requirement of the term "and/or" as used herein. Concurrent applicability of more than one of the options is also understood to fall within the meaning, and therefore satisfy the requirement of the term "and/or."

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integer or step. When used herein the term "comprising" can be substituted with the term "containing" or "including" or sometimes when used herein with the term "having". Any of the aforementioned terms (comprising, containing, including, having), whenever used herein in the context of an aspect or embodiment of the present invention may be substituted with the term "consisting of', though less preferred.

When used herein "consisting of" excludes any element, step, or ingredient not specified in the claim element. When used herein, "consisting essentially of does not exclude materials or steps that do not materially affect the basic and novel characteristics of the claim.

"Administering" or "administration of" a medicament to a patient (and grammatical equivalents of this phrase) refers to direct administration, which may be administration to a patient by a medical professional or may be self-administration, and/or indirect administration, which may be the act of prescribing a drug. E.g., a physician who instructs a patient to self-administer a medicament or provides a patient with a prescription for a drug is administering the drug to the patient.

The term "affibody" refers to a protein that is derived from the Z domain of protein A and that been engineered to bind to a specific target (see Frejd & Kim, 2017. Exp Mol Med. 49(3): e306).

The term "antibody" refers to a molecule comprising at least one immunoglobulin domain that binds to, or is immunologically reactive with, a particular target. The term includes whole antibodies and any antigen binding portion or single chains thereof and combinations thereof; for instance, the term "antibody" in particular includes bivalent antibodies and bivalent bispecific antibodies.

A typical type of antibody comprises at least two heavy chains ("HC") and two light chains ("LC") interconnected by disulfide bonds.

The term "domain" is referred herein to a part of a protein that has a specific function in the protein and that, usually, folds independently.

The term "fusion protein" refers to a protein that has been created through the joining of two or more proteins, or two or more domains. The term "fusion protein" is synonymous to "chimeric protein".

Each "heavy chain" comprises a "heavy chain variable domain" (abbreviated herein as "VH") and a "heavy chain constant domain" (abbreviated herein as "CH"). The heavy chain constant domain typically comprises three constants domains, CH1, CH2, and CH3.

Each "light chain" comprises a "light chain variable domain" (abbreviated herein as "VL") and a "light chain constant domain" ("CL"). The light chain constant domain (CL) can be of the kappa type or of the lambda type. The VH and VL domains can be further subdivided into regions of hypervariability, termed Complementarity Determining Regions ("CDR"), interspersed with regions that are more conserved, termed "framework regions" ("FW").

Each VH and VL is composed of three CDRs and four FWs, arranged from amino-terminus to carboxy-terminus in the following order: FW1, CDR1, FW2, CDR2, FW3, CDR3, FW4. The present disclosure inter alia presents VH and VL sequences as well as the subsequences corresponding to CDR1, CDR2, and CDR3.

A person skilled in the art would understand that the sequences of FW1, FW2, FW3 and FW4 are equally disclosed. For a particular VH, FW1 is the subsequence between the N-terminus of the VH and the N-terminus of H-CDR1, FW2 is the subsequence between the C-terminus of H-CDR1 and the N-terminus of H-CDR2, FW3 is the subsequence between the C-terminus of H-CDR2 and the N-terminus of H-CDR3, and FW4 is the subsequence between the C-terminus of H-CDR3 and the C-terminus of the VH. Similarly, for a particular VL, FW1 is the subsequence between the N-terminus of the VL and the N-terminus of L-CDR1, FW2 is the subsequence between the C-terminus of L-CDR1 and the N-terminus of L-CDR2. FW3 is the subsequence between the C-terminus of L-CDR2 and the N-terminus of L-CDR3, and FW4 is the subsequence between the C-terminus of L-CDR3 and the C-terminus of the VL.

The variable domains of the heavy and light chains contain a region that interacts with a binding target, and this region interacting with a binding target is also referred to as an "antigen-binding site" or "antigen binding site" herein. The constant domains of the antibodies can mediate the binding of the antibody to host tissues or factors, including various cells of the immune system (e.g., effector cells) and the first component (C1q) of the classical complement system. Exemplary antibodies of the present disclosure include typical antibodies, but also bivalent fragments and variations thereof such as a F(ab')2.

As used herein, the term "antibody" encompasses intact polyclonal antibodies, intact monoclonal antibodies, bivalent antibody fragments (such as F(ab')2), multispecific antibodies such as bispecific antibodies, chimeric antibodies, humanized antibodies, human antibodies, and any other modified immunoglobulin molecule comprising an antigen binding site.

An antibody can be of any the five major classes (isotypes) of immunoglobulins: IgA, IgD, IgE, IgG, and IgM, or subclasses thereof (e.g. IgG1, IgG2, IgG3, IgG4, IgA1 and IgA2), based on the identity of their heavy-chain constant domains referred to as alpha, delta, epsilon, gamma, and mu, respectively. The different classes of immunoglobulins have different and well known subunit structures and three-dimensional configurations. Antibodies can be naked or conjugated to other molecules such as therapeutic agents or diagnostic agents to form immunoconjugates.

The term "anticalin" refers to a protein that is derived from the lipocalin and that been engineered to bind to a specific target.

The term "antigen-binding fragment" or "Fab" refers to an antibody fragment comprising one constant and one variable domain of each of the heavy and light chain. A Fab fragment may be obtained by digesting an intact monoclonal antibody with papain.

The term "cancer" refers to a group of diseases, which can be defined as any abnormal benign or malignant new growth of tissue that possesses no physiological function and arises from uncontrolled usually rapid cellular proliferation and has the potential to invade or spread to other parts of the body.

The term "chimeric antigen receptor" or "CAR" refers to a synthetic receptor that targets T cells to a chosen antigen and reprograms T cell function, metabolism and persistence.

"Combination therapy", "in combination with" or "in conjunction with" as used herein denotes any form of concurrent, parallel, simultaneous, sequential or intermittent treatment with at least two distinct treatment modalities (i.e., compounds, components, targeted agents or therapeutic agents). As such, the terms refer to administration of one treatment modality before, during, or after administration of the other treatment modality to the subject. The modalities in combination can be administered in any order. The therapeutically active modalities are administered together (e.g., simultaneously in the same or separate compositions, formulations or unit dosage forms) or separately (e.g., on the same day or on different days and in any order as according to an appropriate dosing protocol for the separate compositions, formulations or unit dosage forms) in a manner and dosing regimen prescribed by a medical care taker or according to a regulatory agency. In general, each treatment modality will be administered at a dose and/or on a time schedule determined for that treatment modality. Optionally, three or more modalities may be used in a combination therapy. Additionally, the combination therapies provided herein may be used in conjunction with other types of treatment. For example, other anti-cancer treatment may be selected from the group consisting of chemotherapy, surgery, radiotherapy (radiation) and/or hormone therapy, amongst other treatments associated with the current standard of care for the subject.

A "complete response" or "complete remission" or "CR" indicates the disappearance of all target lesions as defined in the RECIST v1.1 guideline. This does not always mean the cancer has been cured.

The term "costimulatory signaling domain" refers to a signaling moiety that provides to T cells a signal which, in addition to the primary signal provided by for instance the CD3ζ chain of the TCR/CD3 complex, mediates a T cell response, including, but not limited to, activation, proliferation, differentiation, cytokine secretion, and the like. A co-stimulatory domain can include all or a portion of, but is not limited to, CD27, CD28, 4-1BB (CD137), OX40 (CD134), CD30, CD40, 1COS, lymphocyte function-associated antigen-1 (LFA-1), CD2, CD7, LIGHT, NKG2C, B7-H3, and a ligand that specifically binds with CD83. In some embodiments, the co-stimulatory signaling domain is an intracellular signaling domain that interacts with other intracellular mediators to mediate a cell response including activation, proliferation, differentiation and cytokine secretion, and the like.

The term "designed ankyrin repeat proteins" or "DARPin" refers to a protein that is derived from an ankyrin repeat that has been engineered to bind to a specific target (see Plückthun, 2015. Annu Rev Pharmacol Toxicol. 55:489-511).

"Disease free survival" (DFS) refers to the length of time during and after treatment that the patient remains free of disease.

As used herein, the term "effective amount" of an agent, e.g., a therapeutic agent such as a CART, is that amount sufficient to effect beneficial or desired results, for example, clinical results, and, as such, an "effective amount" depends upon the context in which it is being applied. For example, in the context of administering a therapeutic agent that treats T-ALL, an effective amount can reduce the number of cancer cells; reduce the tumor size or burden; inhibit (i.e., slow to some extent and in a certain embodiment, stop) cancer cell infiltration into peripheral organs; inhibit (i.e., slow to some extent and in a certain embodiment, stop) tumor metastasis; inhibit, to some extent, tumor growth; relieve to some extent one or more of the symptoms associated with the cancer; and/or result in a favorable response such as increased progression-free survival (PFS), disease-free survival (DFS), or overall survival (OS), complete response (CR), partial response (PR), or, in some cases, stable disease (SD), a decrease in progressive disease (PD), a reduced time to progression (TTP) or any combination thereof. The term "effective amount" can be used interchangeably with "effective dose," "therapeutically effective amount," or "therapeutically effective dose".

The term "fynomer" refers to a protein that is derived from the SH3 domain of human Fyn kinase that has been engineered to bind to a specific target (see Bertschinger et al., 2007. Protein Eng Des Sel. 20(2):57-68).

The terms "individual", "patient" or "subject" are used interchangeably in the present application to designate a human being and are not meant to be limiting in any way. The "individual", "patient" or "subject" can be of any age, sex and physical condition. The term "patient in need thereof" usually refers to a patient who suffers from a CD1a-positive cancer.

"Infusion" or "infusing" refers to the introduction of a therapeutic agent-containing solution into the body through a vein for therapeutic purposes. Generally, this is achieved via an intravenous bag.

"Intracellular signaling domain" as used herein refers to all or a portion of one or more domains of a molecule (here the chimeric receptor molecule) that provides for activation of a lymphocyte. Intracellular domains of such molecules mediate a signal by interacting with cellular mediators to result in proliferation, differentiation, activation and other effector functions. Examples of intracellular signaling domains for use in a CAR of the invention include the intracellular sequences of the CD3ζ chain, and/or co-receptors that act in concert to initiate signal transduction following CAR engagement, as well as any derivative or variant of these sequences and any synthetic sequence that has the same functional capability. T cell activation can be said to be mediated by two distinct classes of cytoplasmic signaling sequence: those that initiate antigen-dependent primary activation and provide a T cell receptor like signal (primary cytoplasmic signaling sequences) and those that act in an antigen- independent manner to provide a secondary or co-stimulatory signal (secondary cytoplasmic signaling sequences). Primary cytoplasmic signaling sequences that act in a stimulatory manner may contain signaling motifs which are known as receptor tyrosine-based activation motifs or ITAMs. Examples of ITAM containing primary cytoplasmic signaling sequences include those derived from CD3ζ, FcRy, CD3γ, CD3δ, CD3ε, CD5, CD22, CD79a, CD79b, and CD66d.

The term "monobody" refers to a protein that is derived from a fibronectin type III domain that has been engineered to bind to a specific target (see Koide et al., 2013. J Mol Biol. 415(2): 393-405).

The term "nanobody" refers to a protein comprising the soluble single antigen-binding V-domain of a heavy chain antibody, preferably a camelid heavy chain antibody (see Bannas et al., 2017. Front Immunol. 8:1603).

"Overall Survival" (OS) refers to the time from patient enrollment to death or censored at the date last known alive. OS includes a prolongation in life expectancy as compared to naive or untreated individuals or patients. Overall survival refers to the situation wherein a patient remains alive for a defined period of time, such as one year, five years, etc., e.g., from the time of diagnosis or treatment.

A "partial response" or "PR" refers to at least a 30% decrease in the sum of diameters of target lesions, taking as reference the baseline sum diameter, in response to treatment, as defined in the RECIST v1.1 guideline.

The term "peptide aptamer" refers to a short, 5-20 amino acid residue sequence that can bind to a specific target. Peptide aptamers are typically inserted within a loop region of a stable protein scaffold (see Reverdatto et al., 2015. Curr Top Med Chem. 15(12):1082-101).

As used herein, "pharmaceutically acceptable carrier" or "pharmaceutically acceptable diluent" means any and all solvents, dispersion media, coatings, antibacterial and antifungal agents, isotonic and absorption delaying agents, compatible with pharmaceutical administration. The use of such media and agents for pharmaceutically active substances is well known in the art. Acceptable carriers, excipients, or stabilizers are nontoxic to recipients at the dosages and concentrations employed and, without limiting the scope of the present invention, include: additional buffering agents; preservatives; co-solvents; antioxidants, including ascorbic acid and methionine; chelating agents such as EDTA; metal complexes (e.g., Zn-protein complexes); biodegradable polymers, such as polyesters; salt-forming counterions, such as sodium, polyhydric sugar alcohols; amino acids, such as alanine, glycine, glutamine, asparagine, histidine, arginine, lysine, ornithine, leucine, 2-phenylalanine, glutamic acid, and threonine; organic sugars or sugar alcohols, such as lactitol, stachyose, mannose, sorbose, xylose, ribose, ribitol, myoinisitose, myoinisitol, galactose, galactitol, glycerol, cyclitols (e.g., inositol), polyethylene glycol; sulfur containing reducing agents, such as glutathione, thioctic acid, sodium thioglycolate, thioglycerol, [alpha]-monothioglycerol, and sodium thiosulfate; low molecular weight proteins, such as human serum albumin, bovine serum albumin, gelatin, or other immunoglobulins; and hydrophilic polymers, such as polyvinylpyrrolidone. Other pharmaceutically acceptable carriers, excipients, or stabilizers, such as those described in Remington's Pharmaceutical Sciences 16th edition, Osol, A. Ed. (1980) may also be included in a pharmaceutical composition described herein, provided that they do not adversely affect the desired characteristics of the pharmaceutical composition.

"Progressive disease" or "disease that has progressed" refers to the appearance of one more new lesions or tumors and/or the unequivocal progression of existing non-target lesions as defined in the RECIST v1.1 guideline. Progressive disease or disease that has progressed can also refer to a tumor growth of more than 20 percent since treatment began, either due to an increase in mass or in spread of the tumor.

"Progression free survival" (PFS) refers to the time from enrollment to disease progression or death. PFS is generally measured using the Kaplan-Meier method and Response Evaluation Criteria in Solid Tumors (RECIST) 1.1 standards. Generally, progression free survival refers to the situation wherein a patient remains alive, without the cancer getting worse.

The term "RECIST" means Response Evaluation Criteria in Solid Tumours. RECIST guideline, criteria, or standard, describes a standard approach to solid tumor measurement and definitions for objective assessment of change in tumor size for use in adult and pediatric cancer clinical trials. RECIST v1.1 means version 1.1 of the revised RECIST guideline and it is published in European Journal of Cancers 45 (2009) 228-247.

The term "repebody" refers to a protein that is derived from a leucine-rich repeat module and that been engineered to bind to a specific target (see Lee et al., 2012. PNAS. 109(9): 3299-3304).

The term "respond favorably" generally refers to causing a beneficial state in a subject. With respect to cancer treatment, the term refers to providing a therapeutic effect on the subject. Positive therapeutic effects in cancer can be measured in a number of ways (See, Weber, 2009. J Nucl Med. 50 Suppl 1:1S-10S). For example, tumor growth inhibition, molecular marker expression, serum marker expression, and molecular imaging techniques can all be used to assess therapeutic efficacy of an anti-cancer therapeutic. A favorable response can be assessed, for example, by increased progression-free survival (PFS), disease-free survival (DFS), or overall survival (OS), complete response (CR), partial response (PR), or, in some cases, stable disease (SD), a decrease in progressive disease (PD), a reduced time to progression (TTP) or any combination thereof.

The term "sequence identity" refers to a percentage value obtained when two sequences are compared using a pairwise sequence alignment tool. In the present case, the sequence identity is obtained using the global alignment tool "EMBOSS Needle" using the default settings (Rice et al., 2000. Trends Genet. 16(6):276-7; Li et al., 2015. Nucleic Acids Res. 43(W1):W580-4). The global alignment tool is available at: https://www.ebi.ac.uk/Tools/psa/ .

The term "single-chain antigen-binding fragment" or "scFab" refers to a fusion protein comprising one variable and one constant domain of the light chain of an antibody attached to one variable and one constant domain of the heavy chain of an antibody, wherein the heavy and light chains are linked together through a short peptide.

The term "single-chain variable fragment" or "scFv" refers to a fusion protein comprising the variable domains of the heavy chain and light chain of an antibody linked to one another with a peptide linker. The term also includes a disulfide stabilized Fv (dsFv). Methods of stabilizing scFvs with disulfide bonds are disclosed in Reiter et al., 1996. Nat Biotechnol. 14(10):1239-45.

"Stable disease" refers to disease without progression or relapse as defined in the RECIST v1.1 guideline. In stable disease there is neither sufficient tumor shrinkage to qualify for partial response, nor sufficient tumor increase to qualify as progressive disease.

"Time to Tumor Progression" (TTP) is defined as the time from enrollment to disease progression. TTP is generally measured using the RECIST v1.1 criteria.

### DESCRIPTION OF THE EMBODIMENTS

### The fusion protein of the invention

Currently, the most promising and/or advanced CAR-T cell products on the market or under testing are autologous (made with same patient-derived T lymphocytes) to avoid severe alloimmune rejection due to a mismatch of MHC between the donor and the recipient. Alternatively, universal CAR-T cell therapy would consist of allogenic CAR-T cells that are taken from healthy donors and that have the same killing mechanisms, but better manufacturing processes, cost, safety and applicability. However, two main aspects need to be considered at the time of producing universal CART-T cells. On the one hand, the graft-versus-host disease (GVHD), caused by hyperactivation and induced-cell death resulting from the recognition of the HLA of the recipient tissues cells by the TCR of the CAR-T cells. On the other hand, since the cells infused are T cells, the opposite case of host-versus-graft reaction (HVG) can also occur, where the TCR of the host cells recognise the foreign HLA molecules on the CAR-T cells. Therefore, strategies aimed at reducing the immune reaction between recipient and donors should address not only the hyperactivation of the HLA but also of the TCR.

As explained above, the US6 protein is capable of reducing the expression of MHC-I on the surface of the cell. As also explained above, CD3 molecule is essential to have a functional TCR. Based on this, the authors of the present invention combined the US6 protein with ScFvs against CD3, generating therapeutic fusion proteins that are useful in CAR-T cell therapy.

As shown in Examples 1-2, two fusion proteins comprising a ScFv against CD3e molecule and a US6 protein were generated with the objective to hijack CD3 and HLA inside the cell, avoiding their surface expression (see Figs 2, 3 and 4). Said cells are unable to have a functional TCR (due to the retention of CD3e caused by the presence of an ScFv anti-CD3e bound to US6) but are also unable to have functional HLA (due to the presence of US6 which itself disrupts epitope loading to the HLA in the endoplasmic reticulum). The allogenic T cell reactivity was reduced when the cells comprise said fusion protein, as depicted in Figs. 5 and 6. When the fusion proteins were tested in vivo, as shown in Fig. 7 and 8, it can be observed that cells expressing the fusion proteins preclude to induce a graft versus host disease on mice. Thus, cells transformed with and expressing the fusion proteins designed herein are suitable for allogenic transplants.

To demonstrate that the allogenic cells expressing the fusion proteins of the invention were still cytotoxic and could be used in therapeutic applications, they were transfected with the fusion protein and with a CAR, and thus universal CAR-T cells (called herein CAR-NexTCell) were generated. Example 4 and Figs. 9-13 show the feasibility of this strategy by using three different CARs: a CAR against BCMA, a CAR against CD19, and a CAR against SARS-COV-2.

Said uCAR-T expressing the fusion protein and the CAR still targeted tumour-derived antigens due to its CAR, but will not trigger GVHD nor HVG reaction due to the reduced expression (or even lack of expression) of HLA and TCR on their surface.

In view of the above, **a first aspect** of the present invention refers to a fusion protein, also referred herein as the "fusion protein of the invention", comprising at least two domains, wherein:
a) the first domain comprises a moiety that targets the TCR or HLA, prefrably a CD3 targeting moiety, and
b) the second domain comprises the US6 protein, or a protein with at least 80%, 85%, 86%, 87%, 88%, 89%, 90%, 91%, 92%, 93%, 94%, 95%, 96%, 97%, 98%, 99% or 100% sequence identity to the US6 protein.

In an embodiment, the first domain is placed in the N-terminus of the fusion protein, and the second domain is placed in the C- terminus of the fusion protein. In an embodiment, the first domain is placed in the N-terminus of the second domain.

In an embodiment, a signal peptide is placed in the N-terminus of the fusion protein, preferably in the N-terminus of the first domain. In an embodiment, a linker peptide is placed between the first and the second domain, thereby providing the fusion protein with some flexibility. In an embodiment, the first and second domains are contiguous, with no linker peptide in between.

In an embodiment, the fusion protein only comprises, or consists of, the two domains defined herein. In an embodiment, the fusion protein is an isolated protein. In an embodiment, the fusion protein is a recombinantly produced protein. Methods to produce recombinant proteins are known in the art.

Each of the domains is described in detail below:

### a) the first domain

In an embodiment, the first domain targets the TCR or HLA, including any of their subunits, thereby causing a downregulation or reduction of their expression or function.

The TCR, a defining structure of T cells, is a transmembrane heterodimer consisting of either an alpha and beta chain or delta and gamma chain linked by a disulfide bond. Within these chains are complementary determining regions (CDRs) which determine the antigen to which the TCR will bind. In the TCR, the TCRα and TCRβ subunits (or TCRγ and TCRδ in γδ T cells) are responsible for the recognition of the Major histocompatibility complex(MHC)/antigen ligand. Efficient downregulation of TCR will significantly suppress the TCR-mediated immune attack and reduce GVHD during allogeneic transfusion of T cells. Therefore, in an embodiment, the first domain causes the suppression or downregulation of TCRα and TCRβ subunits (or TCRγ and TCRδ in γδ T cells) or HLA proteins.

In an embodiment, the first domain that targets TCR or HLA affinity molecules is an antibody fragment that specific recognizes and binds to the constant region of TCR, preferably αβTCR, or HLA I.

In an embodiment, the first domain comprises or consists of a CD3 targeting moiety. Preferably, the targeting moiety is a CD3γ (CD3gamma), CD3δ (CD3delta), or CD3ε (CD3epsilon, also called herein CD3e) targeting moiety. Most preferably the targeting moiety is a CD3e targeting moiety.

The term "CD3" or "cluster of differentiation 3" is a protein complex and T cell co-receptor that is involved in activating both the cytotoxic T cell (CD8+ naive T cells) and T helper cells (CD4+ naive T cells). It is composed of four distinct chains. In mammals, the complex contains a CD3γ chain, a CD3δ chain, and two CD3ε (or CD3e) chains. These chains associate with the T-cell receptor (TCR) and the CD3-zeta (ζ-chain) to generate an activation signal in T lymphocytes. The TCR, CD3-zeta, and the other CD3 molecules together constitute the TCR complex.

The term "CD3 targeting moiety" refers to a substance that is able to bind CD3. The term "CD3e targeting moiety" refers to a substance that is able to bind CD3e.

In an embodiment, the CD3 targeting moiety, preferably CD3e targeting moiety, is an antibody, anticalin, repebody, monobody, preferably scFv, Fab, scFab, affibody, fynomer, DARPin, nanobody, or peptide aptamer that specifically binds to CD3.

"Specific binding" or "specifically binds" refer to an antibody, or a ligand, which recognizes and binds with a binding partner (e.g., a stimulatory tumor antigen) protein present in a sample, but which antibody or ligand does not substantially recognize or bind other molecules in the sample. The skilled person is clearly aware of various experimental procedures that can be used to test binding and binding specificity. Some cross-reaction or background binding may be inevitable in many protein-protein interactions; this is not to detract from the "specificity" of the binding between antibody and epitope. The term "directed against" is also applicable when considering the term "specificity" in understanding the interaction between antibody and epitope.

In an embodiment, the CD3 targeting moiety, preferably CD3e targeting moiety, is a ScFv that comprises a VL and a VH domain. Preferably, the CD3 targeting moiety is the moiety defined in the second aspect of the present invention, described below.

In an embodiment, the CD3e targeting moiety comprises a VL domain and a VH domain, wherein said VL domain comprises LCDR1, LCDR2 and LCDR3 polypeptides and said VH domain comprises HCDR1, HCDR2 and HCDR3 polypeptides, and wherein:
- LCDR1 comprises, consists, or consists essentially of SEQ ID NO: 14, or a sequence with at least 85%, 86%, 87%, 88%, 89%, 90%, 91%, 92%, 93%, 94%, 95%, 96%, 97%, 98%, 99% or 100% sequence identity to SEQ ID NO: 14,
- LCDR2 comprises, consists, or consists essentially of SEQ ID NO: 15, or a sequence with at least 85%, 86%, 87%, 88%, 89%, 90%, 91%, 92%, 93%, 94%, 95%, 96%, 97%, 98%, 99% or 100% sequence identity to SEQ ID NO: 15;
- LCDR3 comprises, consists, or consists essentially of SEQ ID NO: 16, or a sequence with at least 85%, 86%, 87%, 88%, 89%, 90%, 91%, 92%, 93%, 94%, 95%, 96%, 97%, 98%, 99% or 100% sequence identity to SEQ ID NO: 16;
- HCDR1 comprises, consists, or consists essentially of SEQ ID NO: 17, or a sequence with at least 85%, 86%, 87%, 88%, 89%, 90%, 91%, 92%, 93%, 94%, 95%, 96%, 97%, 98%, 99% or 100% sequence identity to SEQ ID NO: 17;
- HCDR2 comprises, consists, or consists essentially of SEQ ID NO: 18, or a sequence with at least 85%, 86%, 87%, 88%, 89%, 90%, 91%, 92%, 93%, 94%, 95%, 96%, 97%, 98%, 99% or 100% sequence identity to SEQ ID NO: 18; and
- HCDR3 comprises, consists, or consists essentially SEQ ID NO: 19, or a sequence with at least 85%, 86%, 87%, 88%, 89%, 90%, 91%, 92%, 93%, 94%, 95%, 96%, 97%, 98%, 99% or 100% sequence identity to SEQ ID NO: 19.

In some embodiments of the first aspect, the CD3e targeting moiety is an antibody, F(ab')2, Fab, preferably scFv, or scFab comprising a VL domain and VH domain, wherein said VL domain comprises LCDR1, LCDR2 and LCDR3 polypeptides and said VH domain comprises HCDR1, HCDR2 and HCDR3 polypeptides, and LCDR1 consists of SEQ ID NO: 14, LCDR2 consists of SEQ ID NO: 15, LCDR3 consists of SEQ ID NO: 16, HCDR1 consists of SEQ ID NO: 17, HCDR2 consists of SEQ ID NO: 18, and HCDR3 consists of SEQ ID NO: 19.

In some embodiments, the CD3e targeting moiety of the first aspect is an antibody, preferably scFv, Fab, or scFab comprising a VL domain and VH domain, wherein the VL domain comprises SEQ ID NO: 11 and the VH domain comprises SEQ ID NO: 12. In some embodiments, the CD3e targeting moiety of the first aspect is an antibody, preferably scFv, Fab, or scFab comprising a VL domain and VH domain, wherein the VL domain consists of SEQ ID NO: 11 and the VH domain consists of SEQ ID NO: 12.

The VL and VH of the CD3e targeting moiety described herein in the first aspect, or any of its embodiments, may be humanized. In the humanization process, the framework regions of the variable region of a targeting moiety may be modified, whereas the CDRs are maintained constant. Therefore, in some embodiments of the first aspect, the CD3e targeting moiety is an antibody, F(ab')2, Fab, preferably scFv, or scFab, comprising a VL and a VH domains, wherein the VL domain comprises a sequence having at least 80%, 81%, 82%, 83%, 84%, 85%, 86%, 87%, 88%, 89%, 90%, 91%, 92%, 93%, 94%, 95%, 96%, 97%, 98%, 99% or 100% sequence identity to SEQ ID NO: 11, wherein the CDRs comprised in said VL are maintained constant (i.e., non variable) and consists of SEQ ID NOs: 14, 15, and 16; and wherein the VH domain comprises a sequence having at least 80%, 81%, 82%, 83%, 84%, 85%, 86%, 87%, 88%, 89%, 90%, 91%, 92%, 93%, 94%, 95%, 96%, 97%, 98%, 99% or 100% sequence identity to SEQ ID NO: 12, wherein the CDRs comprised in said VH are maintained constant and consist of SEQ ID NOs: 17, 18 and 19.

Thus, in an embodiment of the first aspect, the CD3e targeting moiety is an antibody, F(ab')2, Fab, preferably scFv, or scFab, and comprises a VL domain and a VH domain, wherein:
- said VL domain comprises a sequence with at least 80%, 81%, 82%, 83%, 84%, 85%, 86%, 87%, 88%, 89%, 90%, 91%, 92%, 93%, 94%, 95%, 96%, 97%, 98%, 99% or 100% sequence identity to SEQ ID NO: 11, and wherein said VL domain comprises CDR polypeptides consisting of LCDR1 as set forth in SEQ ID NO: 14, LCDR2 as set forth in SEQ ID NO: 15, LCDR3 as set forth in SEQ ID NO: 16, and
- said VH domain comprises a sequence with at least 80%, 81%, 82%, 83%, 84%, 85%, 86%, 87%, 88%, 89%, 90%, 91%, 92%, 93%, 94%, 95%, 96%, 97%, 98%, 99% or 100% sequence identity to SEQ ID NO: 12, and wherein said VH domain comprises CDR polypeptides consisting of HCDR1 as set forth in SEQ ID NO: 17, HCDR2 as set forth in SEQ ID NO: 18, and HCDR3 as set forth in SEQ ID NO: 19 and
preferably, wherein the CD3e targeting moiety is capable of binding to human CD3e, preferably as measured by surface plasmon resonance (SPR).

In an embodiment of the first aspect, the CD3e targeting moiety is an antibody, F(ab')2, Fab, preferably scFv, or scFab, and is capable of binding to human CD3e and comprises a VL and a VH domain, wherein said VL domain comprises LCDR1, LCDR2 and LCDR3 polypeptides and said VH domain comprises HCDR1, HCDR2 and HCDR3 polypeptides, and wherein:
- the VL domain comprises a sequence having at least 80%, 81%, 82%, 83%, 84%, 85%, 86%, 87%, 88%, 89%, 90%, 91%, 92%, 93%, 94%, 95%, 96%, 97%, 98%, 99% or 100% sequence identity to SEQ ID NO: 11, and
- the VH domain comprising a sequence having at least 80%, 81%, 82%, 83%, 84%, 85%, 86%, 87%, 88%, 89%, 90%, 91%, 92%, 93%, 94%, 95%, 96%, 97%, 98%, 99% or 100% sequence identity to SEQ ID NO: 12, and
wherein the LCDR1, 2, and 3 comprised in the VL domain consist of SEQ ID NO: 14, 15 and 16, respectively, and wherein the HCDR1, 2 and 3 comprised in said VH domain consists of SEQ ID NO: 17, 18, and 19, respectively.

Preferably, the CD3e targeting moiety of the first aspect comprises, consists, or consists essentially of SEQ ID NO: 13, or a sequence that has at least 80%, 81%, 82%, 83%, 84%, 85%, 86%, 87%, 88%, 89%, 90%, 91%, 92%, 93%, 94%, 95%, 96%, 97%, 98%, 99% or 100% sequence identity to SEQ ID NO: 13. Most preferably, the CD3e targeting moiety consists of SEQ ID NO: 13.

In an embodiment, the CD3 targeting moiety, preferably CD3e targeting moiety, is a ScFv that comprises a VL and a VH domain. In an embodiment, the CD3e targeting moiety comprises a VL domain and a VH domain, wherein said VL domain comprises LCDR1, LCDR2 and LCDR3 polypeptides and said VH domain comprises HCDR1, HCDR2 and HCDR3 polypeptides, and wherein:
- LCDR1 comprises, consists, or consists essentially of SEQ ID NO: 21, or a sequence with at least 85%, 86%, 87%, 88%, 89%, 90%, 91%, 92%, 93%, 94%, 95%, 96%, 97%, 98%, 99% or 100% sequence identity to SEQ ID NO: 21,
- LCDR2 comprises, consists, or consists essentially of SEQ ID NO: 22, or a sequence with at least 85%, 86%, 87%, 88%, 89%, 90%, 91%, 92%, 93%, 94%, 95%, 96%, 97%, 98%, 99% or 100% sequence identity to SEQ ID NO: 22;
- LCDR3 comprises, consists, or consists essentially of SEQ ID NO: 23, or a sequence with at least 85%, 86%, 87%, 88%, 89%, 90%, 91%, 92%, 93%, 94%, 95%, 96%, 97%, 98%, 99% or 100% sequence identity to SEQ ID NO: 23;
- HCDR1 comprises, consists, or consists essentially of SEQ ID NO: 24, or a sequence with at least 85%, 86%, 87%, 88%, 89%, 90%, 91%, 92%, 93%, 94%, 95%, 96%, 97%, 98%, 99% or 100% sequence identity to SEQ ID NO: 24;
- HCDR2 comprises, consists, or consists essentially of SEQ ID NO: 25, or a sequence with at least 85%, 86%, 87%, 88%, 89%, 90%, 91%, 92%, 93%, 94%, 95%, 96%, 97%, 98%, 99% or 100% sequence identity to SEQ ID NO: 25; and
- HCDR3 comprises, consists, or consists essentially SEQ ID NO: 26, or a sequence with at least 85%, 86%, 87%, 88%, 89%, 90%, 91%, 92%, 93%, 94%, 95%, 96%, 97%, 98%, 99% or 100% sequence identity to SEQ ID NO: 26.

In some embodiments of the first aspect, the CD3e targeting moiety is an antibody, F(ab')2, Fab, preferably scFv, or scFab comprising a VL domain and VH domain, wherein said VL domain comprises LCDR1, LCDR2 and LCDR3 polypeptides and said VH domain comprises HCDR1, HCDR2 and HCDR3 polypeptides, and LCDR1 consists of SEQ ID NO: 21, LCDR2 consists of SEQ ID NO: 22, LCDR3 consists of SEQ ID NO: 23, HCDR1 consists of SEQ ID NO: 24, HCDR2 consists of SEQ ID NO: 25, and HCDR3 consists of SEQ ID NO: 26.

In some embodiments, the CD3e targeting moiety of the first aspect is an antibody, preferably scFv, Fab, or scFab comprising a VL domain and VH domain, wherein the VL domain comprises SEQ ID NO: 6 and the VH domain comprises SEQ ID NO: 7. In some embodiments, the CD3e targeting moiety of the first aspect is an antibody, preferably scFv, Fab, or scFab comprising a VL domain and VH domain, wherein the VL domain consists of SEQ ID NO: 6 and the VH domain consists of SEQ ID NO: 7.

The VL and VH of the CD3e targeting moiety described herein in the first aspect, or any of its embodiments, may be humanized. Therefore, in some embodiments of the first aspect, the CD3e targeting moiety is an antibody, F(ab')2, Fab, preferably scFv, or scFab, comprising a VL and a VH domains, wherein the VL domain comprises a sequence having at least 80%, 81%, 82%, 83%, 84%, 85%, 86%, 87%, 88%, 89%, 90%, 91%, 92%, 93%, 94%, 95%, 96%, 97%, 98%, 99% or 100% sequence identity to SEQ ID NO: 6, wherein the CDRs comprised in said VL are maintained constant (i.e., non variable) and consists of SEQ ID NOs: 21, 22, and 23; and wherein the VH domain comprises a sequence having at least 80%, 81%, 82%, 83%, 84%, 85%, 86%, 87%, 88%, 89%, 90%, 91%, 92%, 93%, 94%, 95%, 96%, 97%, 98%, 99% or 100% sequence identity to SEQ ID NO: 7, wherein the CDRs comprised in said VH are maintained constant and consist of SEQ ID NOs: 24, 25 and 26.

Thus, in an embodiment of the first aspect, the CD3e targeting moiety is an antibody, F(ab')2, Fab, preferably scFv, or scFab, and comprises a VL domain and a VH domain, wherein:
- said VL domain comprises a sequence with at least 80%, 81%, 82%, 83%, 84%, 85%, 86%, 87%, 88%, 89%, 90%, 91%, 92%, 93%, 94%, 95%, 96%, 97%, 98%, 99% or 100% sequence identity to SEQ ID NO: 6, and wherein said VL domain comprises CDR polypeptides consisting of LCDR1 as set forth in SEQ ID NO: 21, LCDR2 as set forth in SEQ ID NO: 22, LCDR3 as set forth in SEQ ID NO: 23, and
- said VH domain comprises a sequence with at least 80%, 81%, 82%, 83%, 84%, 85%, 86%, 87%, 88%, 89%, 90%, 91%, 92%, 93%, 94%, 95%, 96%, 97%, 98%, 99% or 100% sequence identity to SEQ ID NO: 7, and wherein said VH domain comprises CDR polypeptides consisting of HCDR1 as set forth in SEQ ID NO: 24, HCDR2 as set forth in SEQ ID NO: 25, and HCDR3 as set forth in SEQ ID NO: 26 and
preferably, wherein the CD3e targeting moiety is capable of binding to human CD3e, preferably as measured by surface plasmon resonance (SPR).

In an embodiment of the first aspect, the CD3e targeting moiety is an antibody, F(ab')2, Fab, preferably scFv, or scFab, and is capable of binding to human CD3e and comprises a VL and a VH domain, wherein said VL domain comprises LCDR1, LCDR2 and LCDR3 polypeptides and said VH domain comprises HCDR1, HCDR2 and HCDR3 polypeptides, and wherein:
- the VL domain comprises a sequence having at least 80%, 81%, 82%, 83%, 84%, 85%, 86%, 87%, 88%, 89%, 90%, 91%, 92%, 93%, 94%, 95%, 96%, 97%, 98%, 99% or 100% sequence identity to SEQ ID NO: 6, and
- the VH domain comprising a sequence having at least 80%, 81%, 82%, 83%, 84%, 85%, 86%, 87%, 88%, 89%, 90%, 91%, 92%, 93%, 94%, 95%, 96%, 97%, 98%, 99% or 100% sequence identity to SEQ ID NO: 7, and
wherein the LCDR1, 2, and 3 comprised in the VL domain consist of SEQ ID NO: 21, 22, and 23, respectively, and wherein the HCDR1, 2 and 3 comprised in said VH domain consists of SEQ ID NO: 24, 25 and 26, respectively.

Preferably, the CD3e targeting moiety of the first aspect comprises, consists, or consists essentially of SEQ ID NO: 27, or a sequence that has at least 80%, 81%, 82%, 83%, 84%, 85%, 86%, 87%, 88%, 89%, 90%, 91%, 92%, 93%, 94%, 95%, 96%, 97%, 98%, 99% or 100% sequence identity to SEQ ID NO: 27. Most preferably, the CD3e targeting moiety consists of SEQ ID NO: 27.

In some embodiments, the VL and VH domains of the CD3 targeting moieties defined herein, preferably the CD3e targeting moieties, are linked by a peptide linker. In some embodiments, the linker comprises at least 5 amino acids, preferably between 5 and 25, preferably between 10-20 amino acids, most preferably 20 amino acids. Preferably, the amino acid is G. Preferably, the peptide linker comprises, consists or consists essentially of SEQ ID NO: 8, or a sequence that has at least 90%, 91%, 92%, 93%, 94%, 95%, 96%, 97%, 98%, or 100% sequence identity to SEQ ID NO: 8.

In some embodiments, the VL and VH domains of the CD3 targeting moieties defined herein, preferably the CD3e targeting moieties, further comprise a signal peptide that is placed preferably at the N-terminal region of the fusion protein, preferably at the N-terminal region of the CD3 targeting moiety. Preferably, the signal peptide comprises, consists or consists essentially of SEQ ID NO: 9, or a sequence that has at least 90%, 91%, 92%, 93%, 94%, 95%, 96%, 97%, 98%, or 100% sequence identity to SEQ ID NO: 9.

### b) the second domain

As explained above, the fusion protein of the invention comprises a second domain, wherein the second domain comprises or consists of the human CMV US6 protein.

By "human CMV US6 protein" or "US6 protein" is referred herein to a human cytomegalovirus (also known as Human herpesvirus 5 or HHV5) protein that is capable of blocking TAP-mediated peptide transport into the ER and inhibit MHC molecules assembly. Importantly, the definition of "US6 protein", as used herein, also to encompasses functional analogues, variants, chimeras, homologues, chemically modified peptides, and fragments thereof, as long as they maintain the ability of being capable of blocking TAP-mediated peptide transport into the ER and inhibit MHC molecules assembly. The term "homologues" includes US6 proteins from genus *Cytomegalovirus,* including Cytomegalovirus that infects other animals than humans, such as chimpanzee Cytomegalovirus or virus from the family Hominidae. Preferably, the term "Homologue" refers to proteins that have at least 85%, 86%, 87%, 88%, 89%, 90%, 91%, 92%, 93%, 94%, preferably 95%, 96%, 97%, 98% or 99% sequence identity to US6 protein of SEQ ID NO: 2, and that maintain the same function as US6 protein of SEQ ID NO: 2, namely blocking TAP-mediated peptide transport into the ER and inhibit MHC molecules assembly. The term "variant" includes both naturally occurring variations in the protein sequence that differ from the wild-type US6 protein, but which are capable of blocking TAP-mediated peptide transport into the ER and inhibit MHC molecules. This term also includes chemically modified polypeptides which are attached to the termini and/or reactive amino acid side groups.

Preferably, the US6 protein of the second domain of the fusion protein of the invention comprises or consists of SEQ ID NO: 2, or a sequence that has at least 70%, 75%, 80%, 85%, 86%, 87%, 88%, 89%, 90%, 91%, 92%, 93%, 94%, 95%, 96%, 97%, 98%, or 100% sequence identity to SEQ ID NO: 2. Preferably, the second domain consists of SEQ ID NO: 2.

As shown in the examples, two fusion proteins were developed with different ScFvs against CD3. Preferred embodiments of the fusion protein are:
In an embodiment, the fusion protein comprises a first domain and a second domain, wherein:
a) the first domain comprises a CD3e targeting moiety, wherein said CD3e targeting moiety is an ScFv that comprises a VL domain and a VH domain, wherein said VL domain comprises LCDR1, LCDR2 and LCDR3 polypeptides and said VH domain comprises HCDR1, HCDR2 and HCDR3 polypeptides, and LCDR1 consists of SEQ ID NO: 14, LCDR2 consists of SEQ ID NO: 15, LCDR3 consists of SEQ ID NO: 16, HCDR1 consists of SEQ ID NO: 17, HCDR2 consists of SEQ ID NO: 18, and HCDR3 consists of SEQ ID NO: 19, and
b) the second domain comprises the US6 protein from human cytomegalovirus, preferably the US6 protein of SEQ ID NO: 2.

In an embodiment, the fusion protein comprises a first domain and a second domain, wherein:
a) the first domain comprises a CD3e targeting moiety, wherein said CD3e targeting moiety is an ScFv that comprises a VL domain and a VH domain, wherein said VL domain comprises LCDR1, LCDR2 and LCDR3 polypeptides and said VH domain comprises HCDR1, HCDR2 and HCDR3 polypeptides, and LCDR1 consists of SEQ ID NO: 21, LCDR2 consists of SEQ ID NO: 22, LCDR3 consists of SEQ ID NO: 23, HCDR1 consists of SEQ ID NO: 24, HCDR2 consists of SEQ ID NO: 25 and HCDR3 consists of SEQ ID NO: 26, and
b) the second domain comprises the US6 protein from human cytomegalovirus, preferably the US6 protein of SEQ ID NO: 2.

In an embodiment, the fusion protein comprises a first domain and a second domain, wherein:
a) the first domain comprises a CD3e targeting moiety, wherein said CD3e targeting moiety is an ScFv that comprises a VL domain and a VH domain, wherein:
   - said VL domain comprises a sequence with at least 85% sequence identity to SEQ ID NO: 11, and comprises CDR polypeptides consisting of LCDR1 as set forth in SEQ ID NO: 14, LCDR2 as set forth in SEQ ID NO: 15, LCDR3 as set forth in SEQ ID NO: 16, and
   - said VH domain comprises a sequence with at least 85% sequence identity to SEQ ID NO: 12, and comprises CDR polypeptides consisting of HCDR1 as set forth in SEQ ID NO: 17, HCDR2 as set forth in SEQ ID NO: 18, and HCDR3 as set forth in SEQ ID NO: 19; and
b) the second domain comprises the US6 protein from human cytomegalovirus, preferably the US6 protein of SEQ ID NO: 2.

In an embodiment, the fusion protein comprises a first domain and a second domain, wherein:
a) the first domain comprises a CD3e targeting moiety, wherein said CD3e targeting moiety is an ScFv that comprises a VL domain and a VH domain, wherein:
   - said VL domain comprises a sequence with at least 85% sequence identity to SEQ ID NO: 6, and comprises CDR polypeptides consisting of LCDR1 as set forth in SEQ ID NO: 21, LCDR2 as set forth in SEQ ID NO: 22, LCDR3 as set forth in SEQ ID NO: 23, and
   - said VH domain comprises a sequence with at least 85% sequence identity to SEQ ID NO: 7, and comprises CDR polypeptides consisting of HCDR1 as set forth in SEQ ID NO: 24, HCDR2 as set forth in SEQ ID NO: 25, and HCDR3 as set forth in SEQ ID NO: 26; and
b) the second domain comprises the US6 protein from human cytomegalovirus, preferably the US6 protein of SEQ ID NO: 2.

In an embodiment, the fusion protein comprises a first domain and a second domain, wherein:
a) the first domain comprises a CD3e targeting moiety, wherein said CD3e targeting moiety is an ScFv that comprises a VL domain and a VH domain, wherein the VL domain comprises SEQ ID NO: 11 and the VH domain comprises SEQ ID NO: 12; and
b) the second domain comprises the US6 protein from human cytomegalovirus, preferably the US6 protein of SEQ ID NO: 2.

In an embodiment, the fusion protein comprises a first domain and a second domain, wherein:
a) the first domain comprises a CD3e targeting moiety, wherein said CD3e targeting moiety is an ScFv that comprises a VL domain and a VH domain, wherein the VL domain comprises SEQ ID NO: 6 and the VH domain comprises SEQ ID NO: 7; and
b) the second domain comprises the US6 protein from human cytomegalovirus, preferably the US6 protein of SEQ ID NO: 2.

In an embodiment, the fusion protein comprises a first domain and a second domain, wherein:
a) the first domain comprises a CD3e targeting moiety, wherein said CD3e targeting moiety is an ScFv that comprises a VL domain and a VH domain, wherein the VL domain consists of SEQ ID NO: 11 and the VH domain consists of SEQ ID NO: 12; and
b) the second domain comprises the US6 protein from human cytomegalovirus, preferably the US6 protein of SEQ ID NO: 2.

In an embodiment, the fusion protein comprises a first domain and a second domain, wherein:
a) the first domain comprises a CD3e targeting moiety, wherein said CD3e targeting moiety is an ScFv that comprises a VL domain and a VH domain, wherein the VL domain consists of SEQ ID NO: 6 and the VH domain consists of SEQ ID NO: 7; and
b) the second domain comprises the US6 protein from human cytomegalovirus, preferably the US6 protein of SEQ ID NO: 2.

In an embodiment, the fusion protein comprises or consists of SEQ ID NO: 20, or a sequence that has at least 70%, 75%, 80%, 75%, 86%, 87%, 88%, 89%, 90%, 91%, 92%, 93%, 94%, 95%, 96%, 97%, 98%, or 100% sequence identity to SEQ ID NO: 20. In an embodiment, the fusion protein comprises or consists of SEQ ID NO: 28, or a sequence that has at least 70%, 75%, 80%, 75%, 86%, 87%, 88%, 89%, 90%, 91%, 92%, 93%, 94%, 95%, 96%, 97%, 98%, or 100% sequence identity to SEQ ID NO: 28.

### CD3e targeting moiety of the invention

In a second aspect, the present invention provides a CD3e targeting moiety, also called herein "the CD3e targeting moiety of the invention". Preferably, the CD3e targeting moiety of the second aspect is an antibody, anticalin, repebody, monobody, preferably scFv, Fab, scFab, affibody, fynomer, DARPin, nanobody, or peptide aptamer that specifically binds to CD3. In an embodiment, the CD3 targeting moiety, preferably CD3e targeting moiety, is a ScFv that comprises a VL and a VH domain.

In an embodiment, the CD3e targeting moiety of the second aspect comprises a VL domain and a VH domain, wherein said VL domain comprises LCDR1, LCDR2 and LCDR3 polypeptides and said VH domain comprises HCDR1, HCDR2 and HCDR3 polypeptides, and wherein:
- LCDR1 comprises, consists, or consists essentially of SEQ ID NO: 14, or a sequence with at least 85%, 86%, 87%, 88%, 89%, 90%, 91%, 92%, 93%, 94%, 95%, 96%, 97%, 98%, 99% or 100% sequence identity to SEQ ID NO: 14,
- LCDR2 comprises, consists, or consists essentially of SEQ ID NO: 15, or a sequence with at least 85%, 86%, 87%, 88%, 89%, 90%, 91%, 92%, 93%, 94%, 95%, 96%, 97%, 98%, 99% or 100% sequence identity to SEQ ID NO: 15;
- LCDR3 comprises, consists, or consists essentially of SEQ ID NO: 16, or a sequence with at least 85%, 86%, 87%, 88%, 89%, 90%, 91%, 92%, 93%, 94%, 95%, 96%, 97%, 98%, 99% or 100% sequence identity to SEQ ID NO: 16;
- HCDR1 comprises, consists, or consists essentially of SEQ ID NO: 17, or a sequence with at least 85%, 86%, 87%, 88%, 89%, 90%, 91%, 92%, 93%, 94%, 95%, 96%, 97%, 98%, 99% or 100% sequence identity to SEQ ID NO: 17;
- HCDR2 comprises, consists, or consists essentially of SEQ ID NO: 18, or a sequence with at least 85%, 86%, 87%, 88%, 89%, 90%, 91%, 92%, 93%, 94%, 95%, 96%, 97%, 98%, 99% or 100% sequence identity to SEQ ID NO: 18; and
- HCDR3 comprises, consists, or consists essentially SEQ ID NO: 19, or a sequence with at least 85%, 86%, 87%, 88%, 89%, 90%, 91%, 92%, 93%, 94%, 95%, 96%, 97%, 98%, 99% or 100% sequence identity to SEQ ID NO: 19.

In some embodiments of the second aspect, the CD3e targeting moiety is an antibody, F(ab')2, Fab, preferably scFv, or scFab comprising a VL domain and VH domain, wherein said VL domain comprises LCDR1, LCDR2 and LCDR3 polypeptides and said VH domain comprises HCDR1, HCDR2 and HCDR3 polypeptides, and LCDR1 consists of SEQ ID NO: 14, LCDR2 consists of SEQ ID NO: 15, LCDR3 consists of SEQ ID NO: 16, HCDR1 consists of SEQ ID NO: 17, HCDR2 consists of SEQ ID NO: 18, and HCDR3 consists of SEQ ID NO: 19.

In some embodiments, the CD3e targeting moiety of the second aspect is an antibody, preferably scFv, Fab, or scFab comprising a VL domain and VH domain, wherein the VL domain comprises SEQ ID NO: 11 and the VH domain comprises SEQ ID NO: 12. In some embodiments, the CD3e targeting moiety of the first aspect is an antibody, preferably scFv, Fab, or scFab comprising a VL domain and VH domain, wherein the VL domain consists of SEQ ID NO: 11 and the VH domain consists of SEQ ID NO: 12.

The VL and VH of the CD3e targeting moiety described herein in the second aspect, or any of its embodiments, may be humanized. In the humanization process, the framework regions of the variable region of a targeting moiety may be modified, whereas the CDRs are maintained constant. Therefore, in some embodiments of the second aspect, the CD3e targeting moiety is an antibody, F(ab')2, Fab, preferably scFv, or scFab, comprising a VL and a VH domains, wherein the VL domain comprises a sequence having at least 80%, 81%, 82%, 83%, 84%, 85%, 86%, 87%, 88%, 89%, 90%, 91%, 92%, 93%, 94%, 95%, 96%, 97%, 98%, 99% or 100% sequence identity to SEQ ID NO: 11, wherein the CDRs comprised in said VL are maintained constant (i.e., non variable) and consists of SEQ ID NOs: 14, 15, and 16; and wherein the VH domain comprises a sequence having at least 80%, 81%, 82%, 83%, 84%, 85%, 86%, 87%, 88%, 89%, 90%, 91%, 92%, 93%, 94%, 95%, 96%, 97%, 98%, 99% or 100% sequence identity to SEQ ID NO: 12, wherein the CDRs comprised in said VH are maintained constant and consist of SEQ ID NOs: 17, 18 and 19.

Thus, in an embodiment of the second aspect, the CD3e targeting moiety is an antibody, F(ab')2, Fab, preferably scFv, or scFab, and comprises a VL domain and a VH domain, wherein:
- said VL domain comprises a sequence with at least 80%, 81%, 82%, 83%, 84%, 85%, 86%, 87%, 88%, 89%, 90%, 91%, 92%, 93%, 94%, 95%, 96%, 97%, 98%, 99% or 100% sequence identity to SEQ ID NO: 11, and wherein said VL domain comprises CDR polypeptides consisting of LCDR1 as set forth in SEQ ID NO: 14, LCDR2 as set forth in SEQ ID NO: 15, LCDR3 as set forth in SEQ ID NO: 16, and
- said VH domain comprises a sequence with at least 80%, 81%, 82%, 83%, 84%, 85%, 86%, 87%, 88%, 89%, 90%, 91%, 92%, 93%, 94%, 95%, 96%, 97%, 98%, 99% or 100% sequence identity to SEQ ID NO: 12, and wherein said VH domain comprises CDR polypeptides consisting of HCDR1 as set forth in SEQ ID NO: 17, HCDR2 as set forth in SEQ ID NO: 18, and HCDR3 as set forth in SEQ ID NO: 19 and
preferably, wherein the CD3e targeting moiety is capable of binding to human CD3e, preferably as measured by surface plasmon resonance (SPR).

In an embodiment of the second aspect, the CD3e targeting moiety is an antibody, F(ab')2, Fab, preferably scFv, or scFab, and is capable of binding to human CD3e and comprises a VL and a VH domain, wherein said VL domain comprises LCDR1, LCDR2 and LCDR3 polypeptides and said VH domain comprises HCDR1, HCDR2 and HCDR3 polypeptides, and wherein:
- the VL domain comprises a sequence having at least 80%, 81%, 82%, 83%, 84%, 85%, 86%, 87%, 88%, 89%, 90%, 91%, 92%, 93%, 94%, 95%, 96%, 97%, 98%, 99% or 100% sequence identity to SEQ ID NO: 11, and
- the VH domain comprising a sequence having at least 80%, 81%, 82%, 83%, 84%, 85%, 86%, 87%, 88%, 89%, 90%, 91%, 92%, 93%, 94%, 95%, 96%, 97%, 98%, 99% or 100% sequence identity to SEQ ID NO: 12, and
wherein the LCDR1, 2, and 3 comprised in the VL domain consist of SEQ ID NO: 14, 15 and 16, respectively, and wherein the HCDR1, 2 and 3 comprised in said VH domain consists of SEQ ID NO: 17, 18, and 19, respectively.

Preferably, the CD3e targeting moiety of the second aspect comprises, consists, or consists essentially of SEQ ID NO: 13, or a sequence that has at least 80%, 81%, 82%, 83%, 84%, 85%, 86%, 87%, 88%, 89%, 90%, 91%, 92%, 93%, 94%, 95%, 96%, 97%, 98%, 99% or 100% sequence identity to SEQ ID NO: 13. Most preferably, the CD3e targeting moiety consists of SEQ ID NO: 13.

In an embodiment, the CD3e targeting moiety of the second aspect comprises a VL domain and a VH domain, wherein said VL domain comprises LCDR1, LCDR2 and LCDR3 polypeptides and said VH domain comprises HCDR1, HCDR2 and HCDR3 polypeptides, and wherein:
- LCDR1 comprises, consists, or consists essentially of SEQ ID NO: 21, or a sequence with at least 85%, 86%, 87%, 88%, 89%, 90%, 91%, 92%, 93%, 94%, 95%, 96%, 97%, 98%, 99% or 100% sequence identity to SEQ ID NO: 21,
- LCDR2 comprises, consists, or consists essentially of SEQ ID NO: 22, or a sequence with at least 85%, 86%, 87%, 88%, 89%, 90%, 91%, 92%, 93%, 94%, 95%, 96%, 97%, 98%, 99% or 100% sequence identity to SEQ ID NO: 22;
- LCDR3 comprises, consists, or consists essentially of SEQ ID NO: 23, or a sequence with at least 85%, 86%, 87%, 88%, 89%, 90%, 91%, 92%, 93%, 94%, 95%, 96%, 97%, 98%, 99% or 100% sequence identity to SEQ ID NO: 23;
- HCDR1 comprises, consists, or consists essentially of SEQ ID NO: 24, or a sequence with at least 85%, 86%, 87%, 88%, 89%, 90%, 91%, 92%, 93%, 94%, 95%, 96%, 97%, 98%, 99% or 100% sequence identity to SEQ ID NO: 24;
- HCDR2 comprises, consists, or consists essentially of SEQ ID NO: 25, or a sequence with at least 85%, 86%, 87%, 88%, 89%, 90%, 91%, 92%, 93%, 94%, 95%, 96%, 97%, 98%, 99% or 100% sequence identity to SEQ ID NO: 25; and
- HCDR3 comprises, consists, or consists essentially SEQ ID NO: 26, or a sequence with at least 85%, 86%, 87%, 88%, 89%, 90%, 91%, 92%, 93%, 94%, 95%, 96%, 97%, 98%, 99% or 100% sequence identity to SEQ ID NO: 26.

In some embodiments of the second aspect, the CD3e targeting moiety is an antibody, F(ab')2, Fab, preferably scFv, or scFab comprising a VL domain and VH domain, wherein said VL domain comprises LCDR1, LCDR2 and LCDR3 polypeptides and said VH domain comprises HCDR1, HCDR2 and HCDR3 polypeptides, and LCDR1 consists of SEQ ID NO: 21, LCDR2 consists of SEQ ID NO: 22, LCDR3 consists of SEQ ID NO: 23, HCDR1 consists of SEQ ID NO: 24, HCDR2 consists of SEQ ID NO: 25, and HCDR3 consists of SEQ ID NO: 26.

In some embodiments, the CD3e targeting moiety of the second aspect is an antibody, preferably scFv, Fab, or scFab comprising a VL domain and VH domain, wherein the VL domain comprises SEQ ID NO: 6 and the VH domain comprises SEQ ID NO: 7. In some embodiments, the CD3e targeting moiety of the first aspect is an antibody, preferably scFv, Fab, or scFab comprising a VL domain and VH domain, wherein the VL domain consists of SEQ ID NO: 6 and the VH domain consists of SEQ ID NO: 7.

The VL and VH of the CD3e targeting moiety described herein in the second aspect, or any of its embodiments, may be humanized. Therefore, in some embodiments of the second aspect, the CD3e targeting moiety is an antibody, F(ab')2, Fab, preferably scFv, or scFab, comprising a VL and a VH domains, wherein the VL domain comprises a sequence having at least 80%, 81%, 82%, 83%, 84%, 85%, 86%, 87%, 88%, 89%, 90%, 91%, 92%, 93%, 94%, 95%, 96%, 97%, 98%, 99% or 100% sequence identity to SEQ ID NO: 6, wherein the CDRs comprised in said VL are maintained constant (i.e., non variable) and consists of SEQ ID NOs: 21, 22, and 23; and wherein the VH domain comprises a sequence having at least 80%, 81%, 82%, 83%, 84%, 85%, 86%, 87%, 88%, 89%, 90%, 91%, 92%, 93%, 94%, 95%, 96%, 97%, 98%, 99% or 100% sequence identity to SEQ ID NO: 7, wherein the CDRs comprised in said VH are maintained constant and consist of SEQ ID NOs: 24, 25 and 26.

Thus, in an embodiment of the second aspect, the CD3e targeting moiety is an antibody, F(ab')2, Fab, preferably scFv, or scFab, and comprises a VL domain and a VH domain, wherein:
- said VL domain comprises a sequence with at least 80%, 81%, 82%, 83%, 84%, 85%, 86%, 87%, 88%, 89%, 90%, 91%, 92%, 93%, 94%, 95%, 96%, 97%, 98%, 99% or 100% sequence identity to SEQ ID NO: 6, and wherein said VL domain comprises CDR polypeptides consisting of LCDR1 as set forth in SEQ ID NO: 21, LCDR2 as set forth in SEQ ID NO: 22, LCDR3 as set forth in SEQ ID NO: 23, and
- said VH domain comprises a sequence with at least 80%, 81%, 82%, 83%, 84%, 85%, 86%, 87%, 88%, 89%, 90%, 91%, 92%, 93%, 94%, 95%, 96%, 97%, 98%, 99% or 100% sequence identity to SEQ ID NO: 7, and wherein said VH domain comprises CDR polypeptides consisting of HCDR1 as set forth in SEQ ID NO: 24, HCDR2 as set forth in SEQ ID NO: 25, and HCDR3 as set forth in SEQ ID NO: 26 and
preferably, wherein the CD3e targeting moiety is capable of binding to human CD3e, preferably as measured by surface plasmon resonance (SPR).

In an embodiment of the second aspect, the CD3e targeting moiety is an antibody, F(ab')2, Fab, preferably scFv, or scFab, and is capable of binding to human CD3e and comprises a VL and a VH domain, wherein said VL domain comprises LCDR1, LCDR2 and LCDR3 polypeptides and said VH domain comprises HCDR1, HCDR2 and HCDR3 polypeptides, and wherein:
- the VL domain comprises a sequence having at least 80%, 81%, 82%, 83%, 84%, 85%, 86%, 87%, 88%, 89%, 90%, 91%, 92%, 93%, 94%, 95%, 96%, 97%, 98%, 99% or 100% sequence identity to SEQ ID NO: 6, and
- the VH domain comprising a sequence having at least 80%, 81%, 82%, 83%, 84%, 85%, 86%, 87%, 88%, 89%, 90%, 91%, 92%, 93%, 94%, 95%, 96%, 97%, 98%, 99% or 100% sequence identity to SEQ ID NO: 7, and
wherein the LCDR1, 2, and 3 comprised in the VL domain consist of SEQ ID NO: 21, 22 and 23, respectively, and wherein the HCDR1, 2 and 3 comprised in said VH domain consists of SEQ ID NO: 24, 25 and 26.

Preferably, the CD3e targeting moiety of the second aspect comprises, consists, or consists essentially of SEQ ID NO: 27, or a sequence that has at least 80%, 81%, 82%, 83%, 84%, 85%, 86%, 87%, 88%, 89%, 90%, 91%, 92%, 93%, 94%, 95%, 96%, 97%, 98%, 99% or 100% sequence identity to SEQ ID NO: 27. Most preferably, the CD3e targeting moiety consists of SEQ ID NO: 27.

In some embodiments, the VL and VH domains of CD3e targeting moiety defined herein, are linked by a peptide linker. In some embodiments, the linker comprises at least 5 amino acids, preferably between 5 and 25, preferably between 10-20 amino acids, most preferably 20 amino acids. Preferably, the amino acid is G. Preferably, the peptide linker comprises, consists or consists essentially of SEQ ID NO: 8, or a sequence that has at least 90%, 91%, 92%, 93%, 94%, 95%, 96%, 97%, 98%, or 100% sequence identity to SEQ ID NO: 8.

In some embodiments, the VL and VH domains of the CD3e targeting moiety further comprise a signal peptide that is placed preferably at the N-terminal region of the CD3e targeting moiety. Preferably, the signal peptide comprises, consists or consists essentially of SEQ ID NO: 9, or a sequence that has at least 90%, 91%, 92%, 93%, 94%, 95%, 96%, 97%, 98%, or 100% sequence identity to SEQ ID NO: 9.

As shown in Figs. 3 and 4, the two fusion proteins tested were able to reduce CD3 and HLA surface expression. Hence, in an embodiment, the fusion protein is capable of reducing CD3, preferably CD3e, surface expression in a cell. Preferably, the reduction in the CD3, preferably CD3e, surface expression in a cell is a statistically significant reduction. In an embodiment, the fusion protein is capable of reducing MHC, preferably HLA-I, surface expression in a cell. Preferably, the reduction in the MHC, preferably HLA-I, surface expression in a cell is a statistically significant reduction.

By "statistically significant reduced expression of a molecule" is referred herein as the determination by an analyst that the reduction in the expression levels is not explainable by chance alone. Statistical hypothesis testing is the method by which the skilled person makes this determination. This test provides a p-value, which is the probability of observing results as extreme as those in the data, assuming the results are truly due to chance alone. A p-value of 0.1 or lower (preferably 0.05, 0.01, 0.001 or lower) is considered herein to be statistically significant. For example, the increase in the expression of the target molecule in a cell, is statistically significant when a statistical test is performed to compare it to the basal expression levels of a reference cell, preferably a cell not treated with the protein of the present invention, and wherein the resulting p-value of said statistical test is of 0.1 or lower, preferably 0.05, 0.01, 0.001 or lower.

Methods to study the reduction in the cell surface expression of a protein are known in the art, such as flow cytometry. The present invention contains in the Examples section examples of methods to measure whether a cell expresses a protein of interest in its surface.

Preferably, the fusion protein is capable of reducing CD3, preferably CD3e, surface expression in a cell, wherein said reduction is at least a 10%, 20%, 30%, 40%, 50%, 60%, 70%, 80%, 90%, or more reduction as compared to a control cell. Preferably, the fusion protein is capable of reducing MHC, preferably HLA-I, surface expression in a cell, wherein said reduction is at least a 10%, 20%, 30%, 40%, 50%, 60%, 70%, 80%, 90%, or more reduction as compared to a control cell. Preferably, the control cell is a cell that does not express or comprise the fusion protein of the invention. Preferably, the reduction in the expression on the surface of the cell is measured by flow cytometry.

### Nucleic acid of the invention

In a **third aspect,** the present invention provides a nucleic acid, also called the "nucleic acid of the invention", encoding any one of the proteins or targeting moieties disclosed in the first and second aspects, or any of their embodiment.

In some embodiments, the nucleic acid is suitable for transducing or transforming a cell. In some embodiments, the nucleic acid is suitable for transducing or transforming a T cell for use in adoptive immunotherapy.

In some embodiments, the nucleic acid is codon optimized for expression in mammalian cells. Codon optimization methods are known in the art.

The nucleic acid of the present invention may be comprised in a viral vector, such as a γ-retroviral or lentiviral vector, which can be used to transduce or transform a T cell. Preferably, the lentiviral vector is infective but not replicative. Preferably, the lentiviral vector lacks the sequences required for the formation of replication competent lentiviruses.

The nucleic acid may also be inserted into a cell through the use of DNA transposons, RNA transfection or genome editing techniques such as TALEN, ZFN and CRISPR/Cas9.

In some embodiments, the nucleic acid encodes for SEQ ID NO: 20, or a sequence with at least 80%, 81%, 82%, 83%, 84%, 85%, 86%, 87%, 88%, 89%, 90%, 91%, 92%, 93%, 94%, 95%, 96%, 97%, 98%, 99% or 100% sequence identity to SEQ ID NO: 20. In some embodiments, the nucleic acid encodes for SEQ ID NO: 28, or a sequence with at least 80%, 81%, 82%, 83%, 84%, 85%, 86%, 87%, 88%, 89%, 90%, 91%, 92%, 93%, 94%, 95%, 96%, 97%, 98%, 99% or 100% sequence identity to SEQ ID NO: 28.

In some embodiments, the nucleic acid encodes for SEQ ID NO: 13, or a sequence with at least 80%, 81%, 82%, 83%, 84%, 85%, 86%, 87%, 88%, 89%, 90%, 91%, 92%, 93%, 94%, 95%, 96%, 97%, 98%, 99% or 100% sequence identity to SEQ ID NO: 13. In some embodiments, the nucleic acid encodes for SEQ ID NO: 27, or a sequence with at least 80%, 81%, 82%, 83%, 84%, 85%, 86%, 87%, 88%, 89%, 90%, 91%, 92%, 93%, 94%, 95%, 96%, 97%, 98%, 99% or 100% sequence identity to SEQ ID NO: 27.

### Cells of the invention

In a **fourth aspect,** the present invention provides a cell, also called the "cell of the invention", comprising the fusion protein of the first aspect, the CD3e targeting moiety of the second aspect and/or the nucleic acid molecule of the third aspect, or any of their embodiments.

In some embodiments, the cell is a T-cell, preferably a human T cell. In some embodiments, the cell is a naive T cell, memory stem T cell or central memory T cell. It is currently thought that these cells are better suited for adaptive immunotherapy.

In some embodiments, the cell is an autologous T cell. The term "autologous cell" refers to a cell obtained from the same patient that is to be treated using any one of the methods of the present invention. However, preferably, the cell is an allo-tolerant T cell. The term "all-tolerant cell" refers to a cell that has been engineered to decrease the risk of a Graft-versus-host disease response. In some embodiments, this is achieved by genomic editing-mediated deletion of TCR and/or β2-microglobulin. Allo-tolerant cells are known in the art.

In some embodiments, the T cell is a human cell, preferably allo-tolerant human T cell. In some embodiments, the cell is a lymphoid precursor, embryonic stem cell or an induced pluripotent stem cell with the capacity to differentiate into a mature T cell.

In some embodiments, the T cell expresses or comprises the fusion protein of the first aspect, or any of its embodiments. Preferably the T cell expresses or comprises SEQ ID NO: 20, or a sequence with at least 80%, 81%, 82%, 83%, 84%, 85%, 86%, 87%, 88%, 89%, 90%, 91%, 92%, 93%, 94%, 95%, 96%, 97%, 98%, 99% or 100% sequence identity to SEQ ID NO: 20. Preferably the T cell expresses or comprises SEQ ID NO: 28, or a sequence with at least 80%, 81%, 82%, 83%, 84%, 85%, 86%, 87%, 88%, 89%, 90%, 91%, 92%, 93%, 94%, 95%, 96%, 97%, 98%, 99% or 100% sequence identity to SEQ ID NO: 28.

In some embodiments, the T cell expresses or comprises the CD3e targeting moiety of the second aspect, or any of its embodiments. Preferably the T cell expresses or comprises SEQ ID NO: 13, or a sequence with at least 80%, 81%, 82%, 83%, 84%, 85%, 86%, 87%, 88%, 89%, 90%, 91%, 92%, 93%, 94%, 95%, 96%, 97%, 98%, 99% or 100% sequence identity to SEQ ID NO: 13. Preferably the T cell expresses or comprises SEQ ID NO: 27, or a sequence with at least 80%, 81%, 82%, 83%, 84%, 85%, 86%, 87%, 88%, 89%, 90%, 91%, 92%, 93%, 94%, 95%, 96%, 97%, 98%, 99% or 100% sequence identity to SEQ ID NO: 27.

In some embodiments, the T cell comprises or expresses the fusion protein as defined in the first aspect or any of its embodiments, wherein said T cell also comprises or expresses a chimeric antigen receptor (CAR). Said CAR may be directed against any antigen, such as tumour-derived, viral, bacterial, fungi, or any other pathogen's antigens. A T cell expressing or comprising both the fusion protein of the invention and a CAR protein is called herein after "the CAR-T cell of the invention" or "CAR-NexTcells". In this case, the CAR-T cell of the invention is a universal (or allo-tolerant) CAR-T cells.

Preferably, the CAR-T cell of the invention comprises or expresses the fusion protein of the invention and a CAR anti BCMA or a BCMA-targeting moiety. The term "B-cell maturation antigen" (BCMA or BCM), also known as tumor necrosis factor receptor superfamily member 17 (TNFRSF17), is a protein that in humans is encoded by the TNFRSF17 gene. The term "BCMA targeting moiety" refers to a substance that is able to bind BCMA. Within the context of a CAR, a BCMA-targeting moiety targets T cells to a BCMA -positive cell, preferably a cancer cell.

Preferably, the CAR-T cell of the invention comprises or expresses the fusion protein of the invention and a CAR anti CD19 or a CD19 targeting moiety. The term "CD19" refers to a IgSF surface glycoprotein of 95 kDa that is expressed from the earliest stages of B cell development until plasma cell terminal differentiation, when its expression is lost. The term "CD19 targeting moiety" refers to a substance that is able to bind CD19. Within the context of a CAR, a CD19-targeting moiety targets T cells to a CD19-positive cell, preferably a cancer cell.

Preferably, the CAR-T cell of the invention comprises or expresses the fusion protein of the invention and a CAR anti a SARS-CoV-2 protein or a SARS-CoV-2 targeting moiety. The term "SARS-CoV-2 targeting moiety" refers to a substance that is able to bind a protein of the SARS-CoV-2 virus. Preferably, the SARS-CoV-2 protein is the Spike protein of the virus.

Of note, this aspect also includes a population or a plurality of cells, preferably the CAR-T cells, of the invention.

### Pharmaceutical composition of the invention

In a **fifth aspect,** the present invention provides a pharmaceutical composition, also called herein "the pharmaceutical composition of the invention", comprising one or more of:
- the fusion protein of the invention,
- the CD3e targeting moiety of the invention,
- the nucleic acid of the invention, and/or
- the cell of the invention, or a plurality of cells thereof,
and a pharmaceutically acceptable carrier or diluent.

A pharmaceutical composition as described herein may also contain other substances. These substances include, but are not limited to, cryoprotectants, surfactants, antioxidants, and stabilizing agents.

In some embodiments, the cells are formulated by first harvesting them from their culture medium, and then washing and concentrating the cells in a medium and container system suitable for administration (a "pharmaceutically acceptable" carrier) in a therapeutically effective amount.

The pharmaceutical composition may also be formulated to be suitable for administration in humans. Forms of administration include, but are not limited to, intravenously, intraperitoneally, intramuscularly, into the bone marrow, into the lymph node, and /or into cerebrospinal fluid.

### Methods and uses of the invention

In a **sixth aspect,** the present invention provides uses and methods of the fusion protein of the first aspect, the CD3e targeting moiety of the second aspect, the nucleic acid molecule of the third aspect, the cell of the fourth aspect, or the pharmaceutical composition of the fifth aspect, including all of their embodiments, wherein the use is in therapy, or the method is a method of treatment. Hence, the fusion protein of the first aspect, the CD3e targeting moiety of the second aspect, the nucleic acid molecule of the third aspect, the cell of the fourth aspect, or the pharmaceutical composition of the fifth aspect, including all of their embodiments, can be used in the generation of a medicament or as an advanced therapy medicinal product (ATMPs).

In an embodiment, the use is in the preparation of a medicine for treating or preventing tumors when combined with a CAR against said tumour. The tumors include solid tumors and non-solid tumors, wherein the solid tumors include, but are not limited to, lymphoma, kidney tumor, neuroblastoma, germ cell tumor, osteosarcoma, chondrosarcoma, soft tissue sarcoma, liver tumor, Thymoma, lung blastoma, pancreatoblastoma, hemangioma, etc.

In an embodiment, the CAR-T cell of the invention expressing the fusion protein of the invention in combination with a CAR protein are used as universal CAR-T cells that are characterized by having a reduced, preferably statistically reduced, graft-versus-host response (GVHD) and/or host versus graft reaction (HVG). Thus, the use is in a method of immunotherapy aimed at reducing the transplantation rejection of CAR-T cells. Preferably, the CAR-T cell of the invention comprising a CAR and the fusion protein of the invention is used in a method to reduce host-versus-graft reaction (HVG). Preferably, the CAR-T cell of the invention comprising a CAR and the fusion protein of the invention is used in a method to reduce graft-versus-host reaction (GVHD). Preferably, the CAR-T cell of the invention comprising or expressing a CAR and the fusion protein of the invention is used in a method to reduce both the host-versus-graft reaction and the graft-versus-host reaction.

The use according to this aspect may also be in method of treating a cancer comprising administering the CAR T-cells of the present invention as defined above, which are T cells comprising or expressing a CAR targeting moiety and the fusion protein of the invention. The terms "treatment" and "therapy", as used in the present application, refer to a set of hygienic, pharmacological, surgical and/or physical means used with the intent to cure and/or alleviate a disease and/or symptoms with the goal of remediating the health problem. The terms "treatment" and "therapy" include preventive and curative methods, since both are directed to the maintenance and/or reestablishment of the health of an individual or animal. Regardless of the origin of the symptoms, disease and disability, the administration of a suitable medicament to alleviate and/or cure a health problem should be interpreted as a form of treatment or therapy within the context of this application.

Preferably, the CAR-T cell of the invention comprises or expresses the fusion protein of the invention and a CAR, and it is used in a method of treatment by immunotherapy. Preferably, the CAR-T cell of the invention comprises or expresses the fusion protein of the invention and a CAR that targets a tumour antigen or a tumour-targeting moiety, and it is used in the treatment of cancer, wherein said cancer is characterized by expressing said tumour antigen.

Preferably, the CAR-T cell of the invention comprises or expresses the fusion protein of the invention and a CAR anti BCMA or a BCMA-targeting moiety and it is used in the prevention (prophylactic) or treatment of a BCMA-positive cancer. Preferably, the BCMA-positive cancer is multiple myeloma. The term "Multiple myeloma" also known as plasma cell myeloma, is a cancer of plasma cells, a type of white blood cell which normally produces antibodies.

Preferably, the CAR-T cell of the invention comprises or expresses the fusion protein of the invention and a CAR anti CD19 or a CD19-targeting moiety, and it is used in the prevention (prophylactic) or treatment of a CD19-positive cancer. Preferably, the CD19-positive cancer includes lymphoblastic leukaemia, non-Hodgkin's lymphoma or chronic lymphocytic leukaemia or any CD19+ disorder.

Preferably, the CAR-T cell of the invention comprises or expresses the fusion protein of the invention and a CAR anti a SARS-CoV-2 protein or a SARS-CoV-2 targeting moiety, and it is used in the prevention (prophylactic) or treatment of an infection caused by SARS-COV-2.

In some embodiments, the method comprises a combination therapy. In some embodiments, the method comprises further administering an immune checkpoint inhibitor. In some embodiments, the cell or pharmaceutical composition as described herein is administered in combination with chemotherapeutic agents and/or immunosuppressants. In an embodiment, a patient is first treated with a chemotherapeutic agent that inhibits or destroys other immune cells followed by the cell or pharmaceutical composition described herein. In some cases, chemotherapy may be avoided entirely.

Each embodiment disclosed herein is contemplated as being applicable to each of the other disclosed embodiments. Thus, all combinations of the various elements described herein are within the scope of the invention. It should also be understood that, unless clearly indicated to the contrary, in any methods claimed herein that include more than one step or act, the order of the steps or acts of the method is not necessarily limited to the order in which the steps or acts of the method are recited.

### Kit of the present invention

In a **seventh aspect,** the present invention provides a kit comprising one or more of:
- the fusion protein of the invention,
- the CD3e targeting moiety of the invention,
- the nucleic acid of the invention, and/or
- the cell of the invention, or a plurality of cells thereof,
- the pharmaceutical composition of the invention.

The materials described above as well as other materials can be packaged together in any suitable combination as a kit useful for performing, or aiding in the performance of, the use and method defined in the fifth aspect. It is useful if the kit components in a given kit are designed and adapted for use together in the disclosed use or method.

### SEQUENCE LISTING

- **SEQ ID NO: 1 - Viral glycoprotein US3:** MKPVLVLAILAVLFLRLADSVPR-PLDVVVSEIRSAHFRVEENQCWFHMGMLYFKGRMSGNF-TEKHFVNVGIVSQSYMDRLQVSGEQYHHDERGAYFEWNIGGHPVTHTVDMV DITLSTRWGDPKKYAACVPQVRMDYSSQTINWYLQRSMRDDNWGLLFRT-LLVYLFSLVVLVLLTVGVSARLRFI
- **SEQ ID NO: 2 - Viral glycoprotein US6:** MDLLIRLGFLLMCAL-PTPGERSSRDPKTLLSLSPRQQACVPRTKSHRPVCYNDTGDCTDADD-SWKQLGEDFAHQCLQAAKKRPKTHKSRPNDRNLEGRLTCQRVRRLLPCDLDI HPSHRLLTLMNNCVCDGAVWNAFRLIERHGFFAVTLYLCCGITLLVVILALLC-SITYESTGRGIRRCGS
- **SEQ ID NO: 3 - Viral glycoprotein E19K:** MRYMILGLLALAAVCSAAKKVEFKE-PACNVTFKSEANECTTLIKCTTEHEKLII-RHKDKIGKYAVYAIWQPGDTNDYNVTVFQGENRKTFMYKFPFYEMCDITMYM SKQYKLWPPQKCLENTGTFCSTALLITALALVCTLLYLKYKSRRSFIDEKKMP
- **SEQ ID NO: 4 - self-cleaving peptide T2A:** EGRGSLLTCGDVEENPGP
- **SEQ ID NO: 5 - ZsGFP:** MAQSKHGLTKEMTMKYRME-GCVDGHKFVITGEGIGYPFKGKQAINLCVVEGGPLPFAEDIL-SAAFMYGNRVFTEYPQDIVDYFKNSCPAGYTWDRSFLFEDGAVCICNADITVS VEENCMYHESKFYGVNFPADGPVMKKMTDNWEP-SCEKIIPVPKQGILKGDVSMYLLLKDG-GRLRCQFDTVYKAKSVPRKMPDWHFIQHKLTREDRSDAKNQKWHLTEHAIAS GSALP
- **SEQ ID NO: 6 ScFV anti-CD3 VL:** DIVMTQSPAIMSAS-PGEKVTMTCSASSSVSYM NWYQQKSGTSPKRWIYDSSKLAS-GVPARFSGSGSGTSYSLTISSMETEDAATYYCQQWSRNPPTFGGGTKLQITR
- **SEQ ID NO: 7 ScFV anti-CD3 VH:** QVQLQQSGELARPGASVKMSCK-ASGYTFTRSTMHWVKQRPGQGLEWIGYINPSSAY-TNYNQKFKDKATLTADKSSSTAYMQLSSLTSEDSAVYYCASPQVHYDYNGFP YWGQGTLVTVSS
- **SEQ ID NO: 8 - LINKER:** GGGGSGGGGSGGGGSGGGGS
- **SEQ ID NO: 9 - CD8a leader:** MALPVTALLLPLALLLHAARP
- **SEQ ID NO: 10 ScFV anti-CD3:** DIVMTQSPAIMSAS-PGEKVTMTCSASSSVSYM NWYQQKSGTSPKRWIYDSSKLAS-GVPARFSGSGSGTSYSLTISSMETEDAATYYCQQWSRNPPTFGGGTKLQITR GGGGSGGGGSGGGGSGGGGSQVQLQQSGELARPGASVKMSCK-ASGYTFTRST-MHWVKQRPGQGLEWIGYINPSSAYTNYNQKFKDKATLTADKSSSTAYMQLSS LTSEDSAVYYCASPQVHYDYNGFPYWGQGTLVTVSS
- **SEQ ID NO: 11 - ScFV anti-CD3mutated VL:** QIVLTQSPAIMSAS-PGEKVTMTCSASSSVSYM NWYQQKSGTSPKRWIYDSSKLAS-GVPARFSGSGSGTSYSLTISSMEAEDAATYYCQQWSRNPPTFGGGTKLEIKR
- **SEQ ID NO: 12: - ScFV anti-CD3mutated VH:** QVQLQQSGAELAKPGASVKM-SCKASGYTFTRSTMHWVKQRPGQGLEWIGYINPSSAY-TNYNQKFKDKATLTADKSSSTAYMQLSSLTSEDSAVYYCARPQVHYDYNGFP YWGQGTTVTVSS
- **SEQ ID NO: 13 - ScFV anti-CD3mutated:** QIVLTQSPAIMSAS-PGEKVTMTCSASSSVSYMNWYQQKSGTSPKRWIYDSSKLAS-GVPARFSGSGSGTSYSLTISSMEAEDAATYYCQQWSRNPPTFGGGTKLEIKR GGGGSGGGGSGGGGSGGGGSQVQLQQSGAELAKPGASVKMSCK-ASGYTFTRST-MHWVKQRPGQGLEWIGYINPSSAYTNYNQKFKDKATLTADKSSSTAYMQLSS LTSEDSAVYYCARPQVHYDYNGFPYWGQGTTVTVSS
- **SEQ ID NO: 14 - LCDR1:** SASSSVSYMN
- **SEQ ID NO: 15 - LCDR2:** DSSKLAS
- **SEQ ID NO: 16 - LCDR3:** QQWSRNPPT
- **SEQ ID NO: 17 - HCDR1:** GYTFTRSTMH
- **SEQ ID NO: 18 - HCDR2:** YINPSSAYTNYNQKFKD
- **SEQ ID NO: 19 - HCDR3:** PQVHYDYNGFPY
- **SEQ ID NO: 20 - Fusion protein anti-CD3e-US6:** MALPVTALLLPLA-LLLHAARPQIVLTQSPAIMSASPGEKVTMTC-SASSSVSYMNWYQQKSGTSPKRWIYDSSKLASGVPARFSGSGSGTSYSLTIS SMEAE-DAATYYCQQWSRNPPTFGGGTKLEIKRGGGGSGGGGSGGGGSGGGGSQV QLQQSGAELAKPGASVKMSCKASGYTFTRSTMHWVKQRPGQGLE-WIGYINPSSAY-TNYNQKFKDKATLTADKSSSTAYMQLSSLTSEDSAVYYCARPQVHYDYNGFP YWGQGTTVTVSSERSSRDPKTLLSLSPRQQACVPRTKSHRPVCYNDTGDCT-DADDSWKQL-GEDFAHQCLQAAKKRPKTHKSRPNDRNLEGRLTCQRVRRLLPCDLDIHPSHR LLTLMNNCVCDGAVWNAFRLIERHGFFAVTLYLCCGITLLVVILALLCSITYEST-GRGIRRCGS
- **SEQ ID NO: 21 - LCDR1:** SASSSVSYMN
- **SEQ ID NO: 22 - LCDR2:** DTSKLAS
- **SEQ ID NO: 23 - LCDR3:** QQWSSNPFT
- **SEQ ID NO: 24 - HCDR1:** RYTMH
- **SEQ ID NO: 25 - HCDR2:** YINPSRGYTNYNQKFKD
- **SEQ ID NO: 26 - HCDR3:** YYDDHYCLDY
- **SEQ ID NO: 27** - **ScFV anti-CD3:** EVQLQQSGAELARPGASVKMSCKAS-GYTFTRYTMHWVKQRPGQGLE-WIGYINPSRGYTNYNQKFKDKATLTTDKSSSTAYMQLSSLTSEDSAVYYCARY YDDHYCLDYWGQGTTLTVSSAGGGGSGGGGSGGGGSGGGGSQI-VLTQSPAIMSAS-PGEKVTMTCSASSSVSYM N WYQQKSGTSPKRWIYDTSKLASGVPAH FRGSG SGTSYSLTISGMEAEDAATYYCQQWSSNPFTFGSGTKLEINR
- **SEQ ID NO: 28 - Fusion protein anti-CD3e-US6:** MALPVTALLLPLALLLHAAR-PEVQLQQSGAELARPGASVKMSCKAS-GYTFTRYTMHWVKQRPGQGLEWIGYINPSRGYTNYNQKFKDKATLTTDKSSS TAYMQLSSLTSEDSAVYYCARYYDDHYCLDYWGQGTTLTVS-SAGGGGSGGGGSGGGGSGGGGSQIVLTQSPAIMSASPGEKVTMTCSASSS VSYMNWYQQKSGTSPKRWIYDTSKLASGVPAHFRGSGSGTSYSLTISG-MEAE-DAATYYCQQWSSNPFTFGSGTKLEINRERSSRDPKTLLSLSPRQQACVPRTK SHRPVCYNDTGDCTDADDSWKQLGEDFAHQCLQAAKKRPKTHKSRPNDRN-LE-GRLTCQRVRRLLPCDLDIHPSHRLLTLMNNCVCDGAVWNAFRLIERHGFFAVT LYLCCGITLLVVILALLCSITYESTGRGIRRCGS

The invention is described below by the following examples, which must be considered as merely illustrative and in no case limiting of the scope of the present invention.

### EXAMPLES

### EXAMPLE 1: Evaluation of different viral proteins to hijack the expression of HLA-I from the surface of human T cells

### Methods

Viral glycoproteins US3 (Seq. 1) and US6 (Seq. 2) from human cytomegalovirus (HHV-5) and the early glycoprotein E19K (Seq. 3) from human adenovirus (HAdV-2) were selected from the literature for their capacity to interfere with antigen presentation (VIPRs) and hijack the expression of HLA-I on the cell surface. Their protein sequences were obtained from UniProt repository, entry numbers B9VXD7, P14334 and P68978 respectively. The aminoacidic sequences of US3, US6 and E19K were back translated into DNA and optimized for *Homo sapiens* codon usage. For a better characterization, each optimized VIPR sequences was cloned into in our lentiviral backbone plasmid (pCCL-ARI-0001) under the EF1a promoter. Downstream the VIPRs, a self-cleaving peptide T2A (Seq. 4) and the green fluorescent protein ZsGreen sequence (Seq. 5) were cloned (Figure 1 A).

Human T cells isolated from buffy coats were activated and transduced individually with the lentiviral vectors codifying each VIPRs. At day 5 post transduction, HLA-I surface expression was assessed by flow cytometry by measuring the mean fluorescence intensity (MFI) from the transduced T cells (ZsGreen positive population) and compared against the untransfected control. Samples were acquired in AttuneNxT Flow Cytometer. FlowJo software was used to analyse the samples.

### Results

Different proteins such as US3, US6 and E19K have already been described to downregulate class I HLA surface expression in different cell models. Expression of these proteins using lentiviral vectors, allowed us to assess the effect on HLA-I expression on the surface of human T cells by flow cytometry. Transduced T cells could be distinguished from non-transduced (UT) cells due to green fluorescent protein expression (Figure 1 B). Expression of class I HLA was then evaluated on control UT and T cells expressing the viral proteins. The results show that the stable expression of US6 protein is the best VIPR at downregulating HLA-I from the surface of T cells. On the other hand, US3 and E19K lead to a relatively poor intra cellular retention of the HLA-I when compared against the untransduced control (Figure 1 C). Based on these results, we decided to pursue the work only with the US6 protein.

### EXAMPLE 2: Construction of a CD3/TCR and HLA-I dual retention system for T cells - NexTCell

### Methods

An ScFv anti-CD3 was obtained by sequencing an anti-CD3ε hybridoma (clone 33-2A3) from the Immunology department's collection, at Hospital Clinic de Barcelona. The anti-CD3ε single-chain variable fragment (scFv) was reconstructed by connecting the light and heavy variable DNA sequences with a flexible domain sequence (Seq. 8), a CD8a leader (Seq. 9) was placed up-stream of the light variable chain for stabilization and trafficking. Next, a protein structure prediction software was used to remodel the anti-CD3 scFv domain by modifying 8 amino acids and adding one Alanine within the frameworks of the ScFv. The construct was then cloned into the pccl-EF1a-US6-ZsGreen, downstream the EF1a promoter and N-terminal domain of US6 protein (Figure 2A).

Additionally, a second anti-CD3 ScFV was fused to the US6 protein, in a similar construct as the one described in Fig. 2A. Hence, in total, two different fusion proteins were tested.

Human T Cells isolated from buffy coat were transduced with the lentiviral vector. At day 6 post transduction HLA-I and CD3 expression were assessed by flow cytometry. Mean Fluorescence intensity was obtained from the transduced T cell population (ZsGreen positive population) to compare HLA-I and CD3 surface expression against the untransfected control.

To enrich the CD3 negative fraction from untransduced T cells, a magnetic isolation using CD3 microbeads (Miltenyi) was performed. Briefly, Cells were incubated with magnetic beads against CD3 as specified in manufacturer protocol. Afterwards cells were passed through a magnetic column (Miltenyi) that allowed separation of labelled cells from non-labelled cells. In order to assess purification efficiency, the fraction of interest was stained with a mouse anti-human CD3z antibody. Stained cells were acquired with Attune NxT cytometer. Results were analysed with FlowJo software.

### Results

Protein blocker expression systems have already been described. Here we hypothesize that by fusing an anti-CD3 scFv to the N terminal end of the US6 protein, we would also retain for the first time the CD3 protein intracellularly together with the class I HLA, thereby obtaining a cell that not only does not cause host-versus-graft reaction but also does not cause a graft-versus-host rejection. Human T cells were transduced with lentiviral vectors coding for this novel protein coexpressed with a reporter green fluorescent protein (GFP) (Figure 2A). This fusion protein translated into an optimal retention of both, the CD3 and HLA-I on GFP+ cells(Figure 2B-C and 3A). Furthermore, the anti-CD3e-US6 modified T cells (from now on referred as NexTCell), could be enriched using a CD3 magnetic-depletion method (Figure 3 D and E).

The second fusion protein also resulted in optimal retention of both, the CD3 and HLA-I (Figure 4). Since both of them were equally suitable, the following experiments were performed only with the first anti-CD3e-US6 chimeric protein.

### EXAMPLE 3: Functional evaluation of NexTCells in an allogenic system

T cells from one donor can strongly recognise via the T cell receptor (TCR) foreign HLA molecules expressed on cells from another donor, in a process known as allorecognition. This interaction translates into T cell alloreactivity against the recognised cell. In fact, this is one of the mechanisms by which an allogenic transplant can generate an event known as Graft Versus Host Disease, in which the donor T cells attack the recipient healthy tissues, often compromising the recipient's health. This recognition can also occur the other way around where T cells from the patient attack the grafted cells, leading to graft rejection.

### Methods

First, NexTCells were evaluated for their limited capacity to be allo-stimulated by their T cell receptor (TCR) when strongly recognizing a foreign HLA-I. This was evaluated with a mixed lymphocyte reaction (MLR), by co-culturing the NexTCells with allogenic monocyte-derived dendritic cells (moDCs). To achieve that, monocytes isolated from buffy coats from one donor were differentiated into DC by culturing them in RPMI medium supplemented with 400 IU/ml of IL-4 and 250 IU/ml of GM-CSF. After 6 days, moDCs were activated with the addition of IL-1b, TNFa, IL-6 and PGE2 at a 10 ng/uL concentration for 24h. On the other hand, enriched NexTCells from another donor were produced as described in previous sections. The MLR was establish by co-culturing the activated moDC and NexTCell (or non-transduced cells) at 1:5 ratios. The allorecognition was assessed by T cell degranulation and T cell activation. The T cell degranulation was evaluated using the expression of the CD017a marker after 5hrs of co-culture by flowcytometry and the T cell activation was measured by IFNg production after 5 days of co-culture by ELISA.

Alternatively, NextCells were also evaluated for their limited HLA-I-mediated recognition when faced to alloreactive T cells. For this, T cells from one donor were isolated and stained with CTFR as the allogeneic effector cells. On the other hand, enriched NexTCells (or control untransduced cells) were left unstained. Co-culture of CD3e-US6/UTD and allogenic T cells were set at a 1:1 ratio. After 16h of co-culture the activation of the allogeneic effector cells (CTFR+) were evaluated by CD69 and CD154 expression by flow cytometry.

### Results

Here we performed an in vitro MLR assay to assess whether the downregulation of CD3 on the surface of NexTCells precludes their alloreactivity against foreign HLA-I. For that, we established an allogenic co-culture of NexTCells (or UTD control cells) and monocyte derived dendritic cells. We evaluated degranulation of T cells towards allogenic moDCs and IFNg production from T cells after 5 days of co-culture. Our results show that T cells expressing the antiCD3e-US6 chimeric protein have a lower activation in comparison to non-transduced T cells in an allogenic context (Figure 5B and C).

NexTCells were then evaluated whether the intracellular retention of HLA-I could prevent those cells from being allorecognised by the "patient's" T cells and prevent graft rejection.

For that reason, we set a rejection experiment were our non-transduced anti-CD3e-US6 T cells were co-cultured in presence of T cells from an allogenic donor. We assessed activation of the allogenic donor T cells by CD69 and CD154 activation markers after 16h of co-culture. Results indicate that a reduction of class I HLA molecules on the T cell surface is enough to significantly decrease the rejection response from allogenic T cells (Figure 6A and B).

### EXAMPLE 4: Human NexTcells preclude to induce a graft versus host disease in mice

After obtaining in vitro evidence that our NexTcell could decrease alloreactivity, we wanted to evaluate if this data could be translated into an in vivo xenograft versus host disease model (XvHD). Despite of the flaws of the system that could be driven by the unspecific recognition of the human T cell receptor to molecules other than the mice MHC, the system should allow to evaluate in vivo the safety of TCR retention in NexTcells.

### Methods

The XvHD experiment was performed using NSG transgenic mice. NSG transgenic mice (N=4) were injected with 10 million of either non-modified T cells (UT) or 10 million NexTCells by intravenous administration. After administration mice were monitored twice per week evaluating their weight, activity and behaviour following guidelines determined in previous literature. We also extracted blood to evaluate human T cell expansion by total event count using flow cytometry. Mice were killed after reaching maximum XvHD threshold or experimental end point. The spleen, liver and bone marrow from the tibia were harvested and after mechanical disaggregation, cells were stained with an anti-human CD45 antibody for total cell count evaluation by flow cytometry (Figure 8A).

### Results

All mice injected with unmodified T cells developed medium to severe grade XvHD, whereas mice injected with anti-CD3e-US6 T cells showed mild to non-existent symptoms of XvHD (Figure 7B). Monitoring of weight during the experiment revealed weight loss in some individuals from the untransduced T cells group, whereas anti-CD3e-US6 individuals maintained their normal weight (Figure 7C). By end-point, a complete survival of the antiCD3e-US6 group was obtained, whereas three individuals of the UT group had to be killed after attaining the pre-established XvHD symptoms threshold (Figure 7D). Blood analysis of both groups showed a progressive increase in circulating human T cells counts in the UT group, whereas the proliferation of T cells in the anti-CD3e-US6 group was extremely low (Figure 7E). The increase of circulating unmodified T cells was probably due to an expansion mediated by the TCR allorecognition of mice molecules.

After sacrificing the mice, different organs were harvested to analyse human T cell infiltration by flow cytometry (Figure 8). The results obtained demonstrate a higher infiltration and engraftment of human T cells in different mice organs within the UT group compared to the anti-CD3e-US6 group. This difference might be explained by the unspecific allorecognition mediated by the TCR in UT group, leading to more unspecific T cell activation. In turn, this could explain the increase in severity of the xenograft versus host symptomatology such as hunching, skin lesions and decrease in activity.

Considering all the results, we have a good correlation with the in vitro alloreactivity assays. This proves that the retention mediated by anti-CD3e-US6 chimeric protein can avoid the activation of human T cells in an allogenic or xenogeneic context, and thus preventing the appearance of GvHD/XvHD symptomatology.

### EXAMPLE 5: Combination of NexTCells with chimeric antigen receptor (CAR)

### Methods

To evaluate the possibility to generate functional CAR-NexTcells. We co-transudced human T cells with vectors that codified for the anti-CD3e-US6 chimeric protein and an anti-BCMA CAR molecule independently. Both vectors were added at a 1:1 ratio. T cells were then expanded for 9 days, and CD3 and CAR surface expression were evaluated by flow cytometry using an anti-CD3-APC, and an anti-human- F(ab)2-biotionylated antibody followed by an incubation with strepatavidin-BV421 before acquiring the samples by flow cytometer.

Following evaluation of CD3 and CAR surface expression, a cytotoxic assay against the suspension U266 and ARP-1 multiple myeloma cell lines was performed. Cytotoxic effect was tested at 2:1, 1:1 and 0,5:1 Effector to target ratios. Cytotoxicity was measured at 16h post co-culture using a luminescence-based approach.

To further validate our results and to demonstrate that any CAR can be combined with the fusion protein anti-CD3e-US6, we also generated CAR-NexTcells with a CAR against CD19 (also called ARI-0001) (Figure 11) and a CAR against the Spike protein of SARS-CoV-2 virus. The cytotoxicity of the CAR-NexTcells expressing ARI-0001 and the fusion protein anti-CD3e-US6 were tested against suspension NALM6 cells (CD19-positive cells) using a luminescence-based approach. The cytotoxicity of the CAR-NexTcells expressing a CAR against the Spike protein of SARS-CoV-2 virus and the fusion protein anti-CD3e-US6 were tested against adherent 293-Spike cells (SARS-CoV-2 Spike-positive cells) by XCelligence assay.

### Results

The co-transduction of human T cells with two independent lentiviral vectors that codified for the modified anti-CD3e-US6 chimeric protein and an anti-BCMA CAR, proved that NexTcells were able to express a chimeric antigen receptor (Figure 9).

Then we wanted to evaluate whether the CAR-NexTcells had cytotoxic activity against their target and compare against conventional CAR-T cells (transduced only with the corresponding CAR). For that we performed a 16-hours bioluminescence cytotoxicity assay either facing ARP-1 and U266 target cells (BCMA+) or NALM6 target cells (CD19+) to the corresponding CAR-Ts and CAR-NexTcells at different effector to target ratios. Alternatively, adherent target cells were evaluated by Xcelligence for 60 hours co-culture. Results show no significant difference between the CAR-T cells and the CAR-NexTcells at a functional level in any of the systems tested: against BCMA (see Figure 10), against CD19 (see Figure 12), and against SARS-CoV-2 virus (see Figure 13).

## Claims

1. A **fusion protein** comprising a first domain and a second domain, wherein:
a) the first domain comprises a CD3 targeting moiety, and
b) the second comprises the US6 protein from human cytomegalovirus.

2. The fusion protein according to claim 1, wherein the second domain comprises SEQ ID NO: 2.

3. The fusion protein according to any one of claims 1 or 2, wherein the CD3 targeting moiety targets CD3e.

4. The fusion protein according to claim 3, wherein the CD3 targeting moiety targets CD3e and is a ScFv comprising a VL and a VH domains, wherein:
- the VL domain comprises LCDR1, LCDR2 and LCDR3 polypeptides and said VH domain comprises HCDR1, HCDR2 and HCDR3 polypeptides, and wherein LCDR1 consists of SEQ ID NO: 14, LCDR2 consists of SEQ ID NO: 15, LCDR3 consists of SEQ ID NO: 16, HCDR1 consists of SEQ ID NO: 17, HCDR2 consists of SEQ ID NO: 18, and HCDR3 consists of SEQ ID NO: 19, or
- the VL domain comprises LCDR1, LCDR2 and LCDR3 polypeptides and said VH domain comprises HCDR1, HCDR2 and HCDR3 polypeptides, and wherein LCDR1 consists of SEQ ID NO: 21, LCDR2 consists of SEQ ID NO: 22, LCDR3 consists of SEQ ID NO: 23, HCDR1 consists of SEQ ID NO: 24, HCDR2 consists of SEQ ID NO: 25, and HCDR3 consists of SEQ ID NO: 26.

5. The fusion protein according to claim 4, wherein the CD3-targeting moiety that targets CD3e is a ScFv comprising a VL and a VH domains, wherein:
- the VL domain comprises SEQ ID NO: 11 and the VH domain comprises SEQ ID NO: 12, or
- the VL domain comprises SEQ ID NO: 6 and the VH domain comprises SEQ ID NO: 7.

6. The fusion protein according to any one of claims 1 to 5, wherein the fusion protein comprises SEQ ID NO: 20 or 28.

7. A **nucleic acid molecule** encoding for the fusion protein according to any one of claims 1 to 6.

8. A **cell** comprising the fusion protein according to any one of claims 1 to 6, or the nucleic acid according to claim 7.

9. The cell according to claim 8, wherein the cell is a T cell.

10. The cell according to any one of claims 8 or 9, wherein the cell also comprises or expresses a chimeric antigen receptor (CAR) protein.

11. The cell according to claim 10, wherein the CAR is selected from the list consisting of a CAR against-BCMA, a CAR against CD19, and a CAR against the Spike protein of SARS-CoV-2 virus.

12. A **pharmaceutical composition** comprising the fusion protein according to any one of claims 1 to 6, the nucleic acid according to claim 7, or the cell according to any one of claims 8 to 11.

13. The fusion protein according to any one of claims 1 to 6, the nucleic acid according to claim 7, the cell according to any one of claims 8 to 11, or the pharmaceutical composition according to claim 12, for use in medicine.

14. The fusion protein according to any one of claims 1 to 6, the nucleic acid according to claim 7, the cell according to any one of claims 8 to 11, or the pharmaceutical composition according to claim 12, for use as an advanced therapy medicinal product (ATMPs).

15. The fusion protein according to any one of claims 1 to 6, the nucleic acid according to claim 7, the cell according to any one of claims 8 to 11, or the pharmaceutical composition according to claim 12, for use in a treatment by immunotherapy that involves reducing the graft-versus-host disease and/or reducing the host-versus-graft rejection.
